# EUROPEAN PATENT APPLICATION

(11) **EP 4 494 672 A1**
(43) Date of publication of application: **22.01.2025**
(21) Application number: 24189250.4
(22) Date of filing: 17.07.2024
(51) Int. Cl.: A61M 5/142

(54) **MEDICAMENT INFUSION DEVICE**

(30) Priority: 21.07.2023 US 202363514899 P; 20.06.2024 US 202418748767
(71) Applicant: Medtronic MiniMed, Inc., Northridge, CA 91325 (US)
(72) Inventor: Antonio, David C., Northridge, 91325 (US); McLevish, Alford L., Roseville, 55113 (US)
(74) Representative: Zimmermann & Partner Patentanwälte mbB

(57) **Abstract**

The present technology includes, for example, an insertion assembly comprising a frame including a tubular sidewall defining an internal cavity and a slot extending longitudinally along a portion of the sidewall. The insertion assembly further comprises a medical device configured to be inserted into the user's skin and a carrier carrying the medical device and at least partially disposed within the internal cavity of the frame. The carrier can be axially movable within the frame from a first, cocked position to a second extended position proximate. The carrier can include a protrusion disposed within the slot of the frame and configured to slide within the slot when the carrier moves axially within the frame. A ratio of a height of the protrusion to a width of the protrusion is greater than 1.

## Description

### CROSS-REFERENCE TO RELATED APPLICATION

This application claims the benefit of priority to U.S. Provisional Application No. 63/514,899, filed July 21, 2023, which is related to U.S. Provisional Application No. 63/514,924 [Attorney Docket No. A0010449US01], titled MEDICAMENT INFUSION DEVICE, filed July 21, 2023, which is incorporated by reference herein in its entirety.

### TECHNICAL FIELD

The present technology relates generally to medical devices, and more particularly, to medicament infusion devices.

### BACKGROUND

Ambulatory infusion pumps are relatively small, at least substantially self-contained devices that are used to introduce drugs and other infusible substances (collectively "medicament") into users' bodies. Some infusion pumps are configured to be worn on a belt, carried in a clothing pocket, or the like. Other infusion pumps are configured to be adhered to skin in patch-like fashion. Infusion pumps are advantageous in that they may be used to, for example, subcutaneously introduce (or "infuse") medicament on an ongoing or even continuous basis outside of a clinical environment. Infusion pumps are also advantageous in that they greatly reduce the frequency of subcutaneous access events, such as multiple needle-based shots. One example of a medicament that may be introduced by an infusion pump is a liquid formulation of insulin. Other exemplary medicaments that may be introduced by an infusion pump include, but are not limited to, drugs that treat cancers and drugs that suppress the perception of pain.

Many conventional infusion pumps have improved user health and quality of life. Nevertheless, the present inventors have determined that conventional infusion pumps are susceptible to a wide range of improvements.

### SUMMARY

Generally, in some embodiments in accordance with the present technology, an insertion assembly can include a frame comprising a tubular sidewall defining an internal cavity. The frame can have a first end portion configured to be positioned adjacent the skin of a user and a second end portion opposite the first end portion along a longitudinal axis of the frame. The frame can include a slot extending longitudinally along a portion of the sidewall. The insertion assembly can include a medical device configured to be inserted into the user's skin. The insertion assembly can further include a carrier carrying the medical device and at least partially disposed within the internal cavity of the frame. The carrier can be axially movable within the frame from a first, cocked position proximate a second end of the frame to a second extended position proximate the first end portion of the frame. The carrier can include a protrusion disposed within the slot of the frame and configured to slide within the slot when the carrier moves axially within the frame. The protrusion can be bound longitudinally between a top surface and a bottom surface, and can have a height measured between the top and bottom surfaces. The protrusion can be bound laterally between first and second side surfaces that abut the sidewall on either side of the slot. The protrusion can have a width measured between the first and second side surfaces. A ratio of the height of the protrusion to the width of the protrusion can be greater than 1. In some embodiments, the ratio of the height of the protrusion to the width of the protrusion is at least 2. In some embodiments, the ratio of the height of the protrusion to the width of the protrusion is at least 4.

According to some aspects of the technology, the carrier includes a plurality of protrusions spaced apart around a circumference of the carrier and the frame includes a plurality of slots spaced apart around a circumference of the frame. Each of the protrusions can be disposed in a corresponding one of the slots.

In some embodiments, the carrier includes a body portion disposed within the internal cavity of the frame. An outer surface of the body portion can be spaced apart from an inner surface of the frame such that the body portion does not contact the inner surface of the frame when sliding axially therein.

In some examples, the protrusion can terminate at or proximal to a radially outer edge of the slot such that the protrusion does not extend radially outwardly of the slot. In some embodiments, the protrusion extends completely through the slot such that a distal end of the protrusion is radially beyond the slot. In some embodiments, a first portion of the protrusion terminates at or proximal to a radially outer edge of the slot such that the protrusion does not extend radially outward of the slot, and a second portion of the protrusion extends completely through the slot such that a distal end of the protrusion is radially beyond the slot.

According to certain embodiments, the carrier can be axially movable within the frame from the second extended position to a third retracted position proximate the second end portion of the frame. A height of the protrusion as the carrier moves from the first cocked position to the second extended position can be greater than a height of the protrusion as the carrier moves from the second extended position to the third retracted position.

Generally, in some embodiments in accordance with the present technology, an insertion assembly can include a frame comprising a tubular sidewall defining an internal cavity. The frame can have a first end portion configured to be positioned against the skin of a user and a second end portion opposite the first end portion along a longitudinal axis of the frame. The frame can include a slot extending longitudinally along a portion of the sidewall. The insertion assembly can further include a needle and a needle carrier carrying the needle and at least partially disposed within the internal cavity of the frame. The needle carrier can be axially movable within the frame. The needle carrier can include a needle carrier protrusion disposed within the slot of the frame and configured to slide within the slot when the needle carrier moves axially within the frame. The insertion assembly can further include a cannula and a cannula carrier carrying the cannula and at least partially disposed within the internal cavity of the frame. The cannula carrier can be axially movable within the frame. The cannula carrier can include a cannula carrier protrusion disposed within the slot of the frame and configured to slide within the slot when the cannula carrier moves axially within the frame. The cannula carrier protrusion and needle carrier protrusion together can form a sliding interface. The sliding interface can be bound longitudinally between a top surface of the needle carrier protrusion and a bottom surface of the cannula carrier protrusion, and has a height measured between the top and bottom surfaces. The sliding interface can be bound laterally between first and second side surfaces that abut the sidewall on either side of the slot. The sliding interface can have a width measured between the first and second side surfaces. A ratio of the height of the sliding interface to the width of the sliding interface can be greater than 1. In some embodiments, the ratio of the height of the sliding interface to the width of the sliding interface is 2 or more. In some examples, a radial length of the cannula carrier protrusion is greater than a radial length of the needle carrier protrusion.

According to several embodiments, the needle carrier and the cannula carrier are coupled to one another when the insertion assembly is in a pre-insertion state. For example, the cannula carrier can include an upwardly extending arm and a tab extending radially inwardly from a distal end portion of the arm. At least in the pre-insertion state, the tab of the cannula carrier extends over and engages a locking ledge of the needle carrier, thereby securing the needle carrier to a top side of the cannula carrier. In some embodiments, in the pre-insertion state, the cannula carrier is configured to rotate relative to the needle carrier. The needle carrier and cannula carrier can remain coupled to one another as they move axially within the frame during insertion, and the rotational positions of the needle carrier and the cannula carrier can be fixed during insertion. The needle carrier and the cannula carrier can be configured to decouple from one another once the cannula is inserted. In some embodiments, alignment of the arm of the cannula carrier with an opening in the frame enables the arm to move radially outwardly and disengage the locking ledge of the needle carrier, thereby releasing the needle carrier from the cannula carrier.

In some embodiments, the needle carrier includes a body portion disposed within the internal cavity of the frame. An outer surface of the body portion can be spaced apart from an inner surface of the frame such that the body portion does not contact the inner surface of the frame when sliding axially therein.

According to certain embodiments, the cannula carrier includes a body portion disposed within the internal cavity of the frame. An outer surface of the body portion can be spaced apart from an inner surface of the frame such that the body portion does not contact the inner surface of the frame when sliding axially therein.

In several embodiments, the needle carrier protrusion and the cannula carrier protrusion are radially aligned about an insertion axis.

In some embodiments, the needle carrier protrusion comprises a plurality of needle carrier protrusions spaced apart about a circumference of the needle carrier, each configured to slide within a corresponding slot in the frame.

According to some embodiments, the cannula carrier protrusion comprises a plurality of cannula carrier protrusions spaced apart about a circumference of the cannula carrier, each configured to slide within a corresponding slot in the frame.

In several embodiments, the cannula, once inserted, is configured to deliver a medicament to the user.

Generally, in some embodiments in accordance with the present technology, an insertion assembly can include a frame comprising a tubular sidewall defining an internal cavity. The frame can have a first end portion configured to be positioned adjacent the skin of a user and a second end portion opposite the first end portion along a longitudinal axis of the frame. The frame can include a slot extending longitudinally along a portion of the sidewall. The insertion assembly can include a medical device configured to be inserted into the user's skin. The insertion assembly can further include a carrier means for carrying the medical device and at least partially disposed within the internal cavity of the frame. The carrier means can be axially movable within the frame from a first, cocked position proximate a second end of the frame to a second extended position proximate the first end portion of the frame. The carrier means can include a protrusion disposed within the slot of the frame and configured to slide within the slot when the carrier means moves axially within the frame. The protrusion can be bound longitudinally between a top surface and a bottom surface, and can have a height measured between the top and bottom surfaces. The protrusion can be bound laterally between first and second side surfaces that abut the sidewall on either side of the slot. The protrusion can have a width measured between the first and second side surfaces. A ratio of the height of the protrusion to the width of the protrusion can be greater than 1. In some embodiments, the ratio of the height of the protrusion to the width of the protrusion is at least 2. In some embodiments, the ratio of the height of the protrusion to the width of the protrusion is at least 4.

According to some aspects of the technology, the carrier means includes a plurality of protrusions spaced apart around a circumference of the carrier means and the frame includes a plurality of slots spaced apart around a circumference of the frame. Each of the protrusions can be disposed in a corresponding one of the slots.

In some embodiments, the carrier means includes a body portion disposed within the internal cavity of the frame. An outer surface of the body portion can be spaced apart from an inner surface of the frame such that the body portion does not contact the inner surface of the frame when sliding axially therein.

In some examples, the protrusion can terminate at or proximal to a radially outer edge of the slot such that the protrusion does not extend radially outwardly of the slot. In some embodiments, the protrusion extends completely through the slot such that a distal end of the protrusion is radially beyond the slot. In some embodiments, a first portion of the protrusion terminates at or proximal to a radially outer edge of the slot such that the protrusion does not extend radially outward of the slot, and a second portion of the protrusion extends completely through the slot such that a distal end of the protrusion is radially beyond the slot.

According to certain embodiments, the carrier means can be axially movable within the frame from the second extended position to a third retracted position proximate the second end portion of the frame. A height of the protrusion as the carrier means moves from the first cocked position to the second extended position can be greater than a height of the protrusion as the carrier means moves from the second extended position to the third retracted position.

Generally, in some embodiments in accordance with the present technology, an insertion assembly can include a frame comprising a tubular sidewall defining an internal cavity. The frame can have a first end portion configured to be positioned against the skin of a user and a second end portion opposite the first end portion along a longitudinal axis of the frame. The frame can include a slot extending longitudinally along a portion of the sidewall. The insertion assembly can further include a needle and a first carrier means for carrying the needle and at least partially disposed within the internal cavity of the frame. The first carrier means can be axially movable within the frame. The first carrier means can include a first carrier means protrusion disposed within the slot of the frame and configured to slide within the slot when the first carrier means moves axially within the frame. The insertion assembly can further include a cannula and a second carrier means for carrying the cannula and at least partially disposed within the internal cavity of the frame. The second carrier means can be axially movable within the frame. The second carrier means can include a second carrier means protrusion disposed within the slot of the frame and configured to slide within the slot when the second carrier means moves axially within the frame. The second carrier means protrusion and first carrier means protrusion together can form a sliding interface. The sliding interface can be bound longitudinally between a top surface of the first carrier means protrusion and a bottom surface of the second carrier means protrusion, and has a height measured between the top and bottom surfaces. The sliding interface can be bound laterally between first and second side surfaces that abut the sidewall on either side of the slot. The sliding interface can have a width measured between the first and second side surfaces. A ratio of the height of the sliding interface to the width of the sliding interface can be greater than 1. In some embodiments, the ratio of the height of the sliding interface to the width of the sliding interface is 2 or more. In some examples, a radial length of the second carrier means protrusion is greater than a radial length of the first carrier means protrusion.

According to several embodiments, the first carrier means and the second carrier means are coupled to one another when the insertion assembly is in a pre-insertion state. For example, the first carrier means can include a first locking means and the second carrier means can include a second locking means. At least in the pre-insertion state, the second locking means engages the first locking means, thereby securing the first carrier means to the second carrier means. In some embodiments, in the pre-insertion state, the second carrier means is configured to rotate relative to the first carrier means. The first carrier means and second carrier means can remain coupled to one another as they move axially within the frame during insertion, and the rotational positions of the first carrier means and the second carrier means can be fixed during insertion. The first carrier means and the second carrier means can be configured to decouple from one another once the cannula is inserted. In some embodiments, alignment of the second locking means with an opening in the frame allows the second locking means to disengage the first locking means, thereby releasing the first carrier means from the second carrier means.

In some embodiments, the first carrier means includes a body portion disposed within the internal cavity of the frame. An outer surface of the body portion can be spaced apart from an inner surface of the frame such that the body portion does not contact the inner surface of the frame when sliding axially therein.

According to certain embodiments, the second carrier means includes a body portion disposed within the internal cavity of the frame. An outer surface of the body portion can be spaced apart from an inner surface of the frame such that the body portion does not contact the inner surface of the frame when sliding axially therein.

In several embodiments, the first carrier means protrusion and the second carrier means protrusion are radially aligned about an insertion axis.

In some embodiments, the first carrier means protrusion comprises a plurality of first carrier means protrusions spaced apart about a circumference of the first carrier means, each configured to slide within a corresponding slot in the frame.

According to some embodiments, the second carrier means protrusion comprises a plurality of second carrier means protrusions spaced apart about a circumference of the second carrier means, each configured to slide within a corresponding slot in the frame.

In several embodiments, the cannula, once inserted, is configured to deliver a medicament to the user.

Generally, in some embodiments in accordance with the present technology, a medicament infusion device comprises a reservoir assembly comprising a reservoir configured to retain medicament therein. The reservoir can be coupled to a reservoir outlet. The medicament infusion device can further comprise a cannula carrier and an infusion cannula extending downwardly away from the cannula carrier. The cannula can define a cannula lumen and is configured to be inserted into a user's skin. The medicament infusion device can further comprise a seal assembly comprising an annular dock seal configured to engage the cannula carrier such that the dock seal surrounds at least a portion of the cannula carrier. The dock seal can comprise an upper surface, a lower surface, a radially inner surface extending between the upper surface and the lower surface where the radially inner surface configured to abut the cannula carrier, a radially outer surface extending between the upper surface and the lower surface, and a through-hole defining a first fluid flow path between the radially inner surface and the radially outer surface, where the first fluid flow path is configured to be in fluid communication with the cannula lumen when the dock seal engages the cannula carrier. The seal assembly can further comprise a seal housing circumferentially surrounding the dock seal. The seal housing can comprise an upper frame portion comprising an upper flange configured to abut the upper surface of the dock seal, a lower frame portion comprising a lower flange configured to abut the lower surface of the dock seal, an annular side portion extending between the upper frame portion and the lower frame portion, where the side portion configured to abut at least a portion of the radially outer surface of the dock seal to define an annular channel between the side portion of the seal housing and the dock seal, and a second fluid flow path extending through the side portion and configured to be fluidically coupled to the reservoir outlet, where the second fluid flow path is in fluid communication with the first fluid flow path defined by the through-hole of the dock seal.

In some embodiments, the annular dock seal extends circumferentially around a vertical axis and is symmetric about at a horizontal plane that is orthogonal to the vertical axis.

According to some examples, contact between the radially inner surface of the dock seal and a radially outer surface of the cannula carrier defines a second annular channel, the second annular channel in fluid communication with the first fluid flow path and the cannula lumen.

In some embodiments, the dock seal comprises a variable sidewall thickness between the radially inner surface and the radially outer surface. The dock seal can further comprise an intermediate portion having a first sidewall thickness, where the through-hole extends through the intermediate portion between the radially inner surface and the radially outer surface; an upper widened portion disposed axially above the intermediate portion and having a second sidewall thickness greater than the first sidewall thickness, the upper widened portion defining an upper radially inner sealing surface and an upper radially outer sealing surface; and a lower widened portion disposed axially below the intermediate portion and having a third sidewall thickness greater than the first sidewall thickness, the lower widened portion defining a lower radially inner sealing surface and a lower radially outer sealing surface.

According to some examples, the annular channel is defined by the space between the intermediate portion of the dock seal and the side portion of the seal housing.

In some embodiments, a second annular channel is defined by the space between the intermediate portion of the dock seal and a side portion of the cannula carrier.

According to several aspects of the present technology, contact between the upper flange and the upper surface of the dock seal creates a fluid-tight seal.

In several examples, contact between the lower flange and the lower surface of the dock seal creates a fluid-tight seal.

In some embodiments, the cannula carrier comprises a through-hole in fluid communication with the cannula lumen and in fluid communication with the first fluid flow path.

In some examples, the medicament infusion device further comprises an insertion assembly configured to move the cannula carrier between a disengaged configuration in which the cannula carrier does not contact the dock seal and an engaged configuration in which the dock seal surrounds at least a portion of the cannula carrier.

In some embodiments, a lowermost surface of the dock seal abuts the lower flange.

Generally, in some embodiments in accordance with the present technology, a seal assembly for a medicament infusion device comprises an annular dock seal comprising an upper surface, a lower surface, a radially inner surface, a radially outer surface, and a through-hole extending between the radially outer surface and the radially inner surface, where the through-hole defining a first fluid flow path. The seal assembly can further comprise a seal housing at least partially surrounding the dock seal, the seal housing comprising an upper flange configured to contact the upper surface of the dock seal, a lower flange configured to contact the lower surface of the dock seal, a side portion configured to contact at least a portion of the radially outer surface of the dock seal to define an annular channel between the side portion of the seal housing and the radially outer surface of the dock seal, and a second fluid flow path extending through the side portion such that the second fluid flow path is in fluid communication with the first fluid flow path.

In some embodiments, the annular dock seal extends circumferentially around a vertical axis, and wherein the annular dock seal is symmetric about a horizontal plane that is orthogonal to the vertical axis.

In some embodiments, the dock seal comprises a variable sidewall thickness between the radially inner surface and the radially outer surface. The dock seal can further comprise an intermediate portion having a first sidewall thickness, where the through-hole can extend through the intermediate portion between the radially inner surface and the radially outer surface. The dock seal can further comprise an upper widened portion disposed axially above the intermediate portion and having a second sidewall thickness greater than the first sidewall thickness, where the upper widened portion defining an upper radially inner sealing surface and an upper radially outer sealing surface. The dock seal can further comprise a lower widened portion disposed axially below the intermediate portion and having a third sidewall thickness greater than the first sidewall thickness, where the lower widened portion defining a lower radially inner sealing surface and a lower radially outer sealing surface.

According to some embodiments, the annular channel is defined by the space between the intermediate portion of the dock seal and the side portion of the seal housing.

In some examples, contact between the upper flange and the upper surface of the dock seal creates a fluid-tight seal, and contact between the lower flange and the lower surface of the dock seal creates a fluid-tight seal. In several embodiments, contact between the upper flange and the upper surface of the dock seal creates a fluid-tight seal, and contact between the lower flange and the lower surface of the dock seal creates a fluid-tight seal.

In some embodiments, a lowermost surface of the dock seal abuts the lower flange.

Generally, in some embodiments in accordance with the present technology, a dock seal for a medicament infusion device comprises an annular body having a sidewall defining a radially outer surface and a radially inner surface surrounding a central opening configured to receive a cannula carrier therein, where the sidewall has a variable thickness such that the sidewall defines an intermediate portion having a first sidewall thickness; an upper widened portion disposed axially above the intermediate portion and having a second sidewall thickness greater than the first sidewall thickness, the upper widened portion defining an upper radially inner sealing surface and an upper radially outer sealing surface; a lower widened portion disposed axially below the intermediate portion and having a third sidewall thickness greater than the first sidewall thickness, the lower widened portion defining a lower radially inner sealing surface and a lower radially outer sealing surface; and a through-hole extending through the intermediate portion of the sidewall between the radially inner surface and the radially outer surface.

In some embodiments, the annular body extends circumferentially around a vertical axis, and the annular body is symmetric about at a horizontal plane that is orthogonal to the vertical axis.

According to some examples, the upper radially inner sealing surface and the lower radially inner sealing surface each project radially inwardly with respect to the intermediate portion. In several embodiments, the upper radially outer sealing surface and the lower radially inner sealing surface each project radially outwardly with respect to the intermediate portion.

Generally, in some embodiments in accordance with the present technology, a medicament infusion device comprises a reservoir assembly comprising a reservoir configured to retain medicament therein. The reservoir can be coupled to a reservoir outlet. The medicament infusion device can further comprise a cannula carrying means and an infusion cannula extending downwardly away from the cannula carrying means. The cannula can define a cannula lumen and is configured to be inserted into a user's skin. The medicament infusion device can further comprise a seal assembly comprising an annular dock seal configured to engage the cannula carrying means such that the dock seal surrounds at least a portion of the cannula carrying means. The dock seal can comprise an upper surface, a lower surface, a radially inner surface extending between the upper surface and the lower surface where the radially inner surface configured to abut the cannula carrying means, a radially outer surface extending between the upper surface and the lower surface, and a through-hole defining a first fluid flow path between the radially inner surface and the radially outer surface, where the first fluid flow path is configured to be in fluid communication with the cannula lumen when the dock seal engages the cannula carrying means. The seal assembly can further comprise a seal housing circumferentially surrounding the dock seal. The seal housing can comprise an upper frame portion comprising an upper flange configured to abut the upper surface of the dock seal, a lower frame portion comprising a lower flange configured to abut the lower surface of the dock seal, an annular side portion extending between the upper frame portion and the lower frame portion, where the side portion configured to abut at least a portion of the radially outer surface of the dock seal to define an annular channel between the side portion of the seal housing and the dock seal, and a second fluid flow path extending through the side portion and configured to be fluidically coupled to the reservoir outlet, where the second fluid flow path is in fluid communication with the first fluid flow path defined by the through-hole of the dock seal.

In some embodiments, the annular dock seal extends circumferentially around a vertical axis and is symmetric about at a horizontal plane that is orthogonal to the vertical axis.

According to some examples, contact between the radially inner surface of the dock seal and a radially outer surface of the cannula carrying means defines a second annular channel, the second annular channel in fluid communication with the first fluid flow path and the cannula lumen.

In some embodiments, the dock seal comprises a variable sidewall thickness between the radially inner surface and the radially outer surface. The dock seal can further comprise an intermediate portion having a first sidewall thickness, where the through-hole extends through the intermediate portion between the radially inner surface and the radially outer surface; an upper widened portion disposed axially above the intermediate portion and having a second sidewall thickness greater than the first sidewall thickness, the upper widened portion defining an upper radially inner sealing surface and an upper radially outer sealing surface; and a lower widened portion disposed axially below the intermediate portion and having a third sidewall thickness greater than the first sidewall thickness, the lower widened portion defining a lower radially inner sealing surface and a lower radially outer sealing surface.

According to some examples, the annular channel is defined by the space between the intermediate portion of the dock seal and the side portion of the seal housing.

In some embodiments, a second annular channel is defined by the space between the intermediate portion of the dock seal and a side portion of the cannula carrying means.

According to several aspects of the present technology, contact between the upper flange and the upper surface of the dock seal creates a fluid-tight seal.

In several examples, contact between the lower flange and the lower surface of the dock seal creates a fluid-tight seal.

In some embodiments, the cannula carrying means comprises a through-hole in fluid communication with the cannula lumen and in fluid communication with the first fluid flow path.

In some examples, the medicament infusion device further comprises an insertion assembly configured to move the cannula carrying means between a disengaged configuration in which the cannula carrying means does not contact the dock seal and an engaged configuration in which the dock seal surrounds at least a portion of the cannula carrying means.

In some embodiments, a lowermost surface of the dock seal abuts the lower flange.

Generally, in some embodiments in accordance with the present technology, a seal assembly for a medicament infusion device comprises an annular dock seal comprising an upper surface, a lower surface, a radially inner surface, a radially outer surface, and a through-hole extending between the radially outer surface and the radially inner surface, where the through-hole defining a first fluid flow path. The seal assembly can further comprise a seal housing at least partially surrounding the dock seal, the seal housing comprising an upper flange configured to contact the upper surface of the dock seal, a lower flange configured to contact the lower surface of the dock seal, a side portion configured to contact at least a portion of the radially outer surface of the dock seal to define an annular channel between the side portion of the seal housing and the radially outer surface of the dock seal, and a second fluid flow path extending through the side portion such that the second fluid flow path is in fluid communication with the first fluid flow path.

In some embodiments, the annular dock seal extends circumferentially around a vertical axis, and wherein the annular dock seal is symmetric about a horizontal plane that is orthogonal to the vertical axis.

In some embodiments, the dock seal comprises a variable sidewall thickness between the radially inner surface and the radially outer surface. The dock seal can further comprise an intermediate portion having a first sidewall thickness, where the through-hole can extend through the intermediate portion between the radially inner surface and the radially outer surface. The dock seal can further comprise an upper widened portion disposed axially above the intermediate portion and having a second sidewall thickness greater than the first sidewall thickness, where the upper widened portion defining an upper radially inner sealing surface and an upper radially outer sealing surface. The dock seal can further comprise a lower widened portion disposed axially below the intermediate portion and having a third sidewall thickness greater than the first sidewall thickness, where the lower widened portion defining a lower radially inner sealing surface and a lower radially outer sealing surface.

According to some embodiments, the annular channel is defined by the space between the intermediate portion of the dock seal and the side portion of the seal housing.

In some examples, contact between the upper flange and the upper surface of the dock seal creates a fluid-tight seal, and contact between the lower flange and the lower surface of the dock seal creates a fluid-tight seal. In several embodiments, contact between the upper flange and the upper surface of the dock seal creates a fluid-tight seal, and contact between the lower flange and the lower surface of the dock seal creates a fluid-tight seal.

In some embodiments, a lowermost surface of the dock seal abuts the lower flange.

Generally, in some embodiments in accordance with the present technology, a dock seal for a medicament infusion device comprises an annular body having a sidewall defining a radially outer surface and a radially inner surface surrounding a central opening configured to receive a cannula carrying means therein, where the sidewall has a variable thickness such that the sidewall defines an intermediate portion having a first sidewall thickness; an upper widened portion disposed axially above the intermediate portion and having a second sidewall thickness greater than the first sidewall thickness, the upper widened portion defining an upper radially inner sealing surface and an upper radially outer sealing surface; a lower widened portion disposed axially below the intermediate portion and having a third sidewall thickness greater than the first sidewall thickness, the lower widened portion defining a lower radially inner sealing surface and a lower radially outer sealing surface; and a through-hole extending through the intermediate portion of the sidewall between the radially inner surface and the radially outer surface.

In some embodiments, the annular body extends circumferentially around a vertical axis, and the annular body is symmetric about at a horizontal plane that is orthogonal to the vertical axis.

According to some examples, the upper radially inner sealing surface and the lower radially inner sealing surface each project radially inwardly with respect to the intermediate portion. In several embodiments, the upper radially outer sealing surface and the lower radially inner sealing surface each project radially outwardly with respect to the intermediate portion.

Further disclosed herein is that the present technology includes, for example, an insertion assembly comprising a frame including a tubular sidewall defining an internal cavity and a slot extending longitudinally along a portion of the sidewall. The insertion assembly further comprises a medical device configured to be inserted into the user's skin and a carrier carrying the medical device and at least partially disposed within the internal cavity of the frame. The carrier can be axially movable within the frame from a first, cocked position to a second extended position proximate. The carrier can include a protrusion disposed within the slot of the frame and configured to slide within the slot when the carrier moves axially within the frame. A ratio of a height of the protrusion to a width of the protrusion is greater than 1.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A is a perspective view of the top of an infusion device in accordance with several examples of the present technology.
FIG. 1B is a perspective view of the bottom of the infusion device of FIG. 1A.
FIG. 2A is a perspective view of a durable assembly of the infusion device of FIGS. 1A and 1B.
FIGS. 2B and 2C are perspective views of certain components of the durable assembly illustrated in FIG. 2A.
FIG. 3A is a perspective view of a disposable assembly of the infusion device of FIGS. 1A and 1B.
FIG. 3B is a perspective view of certain components of the disposable assembly illustrated in FIG. 3A.
FIG. 4 schematically depicts an insertion assembly of the disposable assembly illustrated in FIG. 3B.
FIG. 5 schematically depicts a seal assembly of the disposable assembly illustrated in FIG. 3B and configured in accordance with the present technology.
FIG. 6A is a perspective view depicting an insertion assembly configured in accordance with the present technology, shown in a pre-insertion state.
FIG. 6B is a section view of the insertion assembly shown in FIG. 6A.
FIG. 7 is an exploded view of the insertion assembly shown in FIGS. 6A and 6B.
FIG. 8 is an isolated view of the needle carrier of the insertion assembly shown in FIGS. 6A, 6B, and 7.
FIG. 9 is an isolated view of the cannula carrier of the insertion assembly shown in FIGS. 6A and 6B.
FIG. 10 is an isolated view of a carrier assembly comprising the needle carrier of FIG. 8 and the cannula carrier of FIG. 9, in accordance with examples of the present technology.
FIG. 11A is a perspective view of the insertion assembly of FIGS. 6A and 6B, shown after insertion has been triggered while the carrier assembly is moving away from the pre-insertion position to the extended position.
FIG. 11B is a top view of the insertion assembly of FIGS. 6A and 6B, showing a portion of a section taken along lines 11B-11B in FIG. 11A.
FIG. 12 is a perspective view of the insertion assembly of FIGS. 6A and 6B, shown as insertion is being triggered by rotation of the cannula carrier.
FIGS. 13A and 13B are perspective and cross-sectional views, respectively, of the insertion assembly of FIGS. 6A and 6B, shown in an extended position.
FIGS. 14A and 14B are perspective and cross-sectional views, respectively, of the insertion assembly of FIGS. 6A and 6B, shown in a retracted position.
FIG. 15 is a side cross-sectional view of a seal assembly in accordance with examples of the present technology, shown coupled to an insertion assembly.
FIG. 16A is a side perspective view of a dock seal in accordance with examples of the present technology.
FIG. 16B is a side cross-sectional view of the dock seal shown in FIG. 16A.
FIG. 17 is a side cross-sectional view of a seal assembly engaged with a cannula carrier in accordance with examples of the present technology.
FIG. 18 is a side cross-sectional view of a portion of a seal assembly engaged with a cannula carrier in accordance with examples of the present technology.

### DETAILED DESCRIPTION

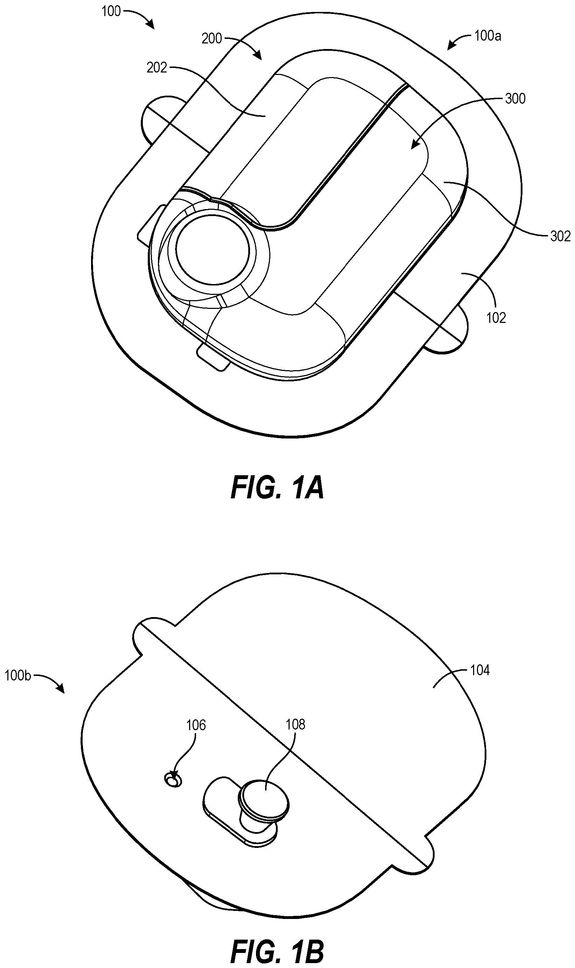

The present technology comprises infusion devices configured to be adhered to the user's skin above the delivery site (sometimes referred to as "patch pumps"). FIGS. 1A and 1B show top and bottom views, respectively, of an infusion device 100 in accordance with several examples of the present technology. A bottom surface 100b of the device 100 is configured to be adhered to the user's skin with the top surface 100a facing away from the user. The device 100 may be a two-piece device including a durable assembly 200 and a disposable assembly 300, each having respective housings 202 and 302. The durable assembly 200 and disposable assembly 300 are disposed on an adhesive pad 102 with adhesive backing 104 for securing to the user's skin. The bottom surface 100b of the device 100 may also include a pull-before-use plug (PBUP) 108 and a fill port 106. While a two-piece device is shown as an example, in other exemplary devices, the device 100 may be a single piece, fully disposable unit.

To use the infusion device 100, the user (e.g., the patient) connects the disposable assembly 300 to the durable assembly 200. Unless the reservoir of the disposable assembly 300 has been sufficiently pre-loaded, the user injects a desired amount of medicament into the reservoir via the fill port 106. A plunger seek procedure may be initiated, either by the user or automatically. To adhere the device 100 to the user, the adhesive backing 104 may be peeled off to expose adhesive on the adhesive pad 102; the PBUP 108 may be removed; and/or the device 100 may be positioned over the chosen body location and pressed gently to adhere the adhesive pad 102 to the skin surface. In some examples, plunger seek is not required.

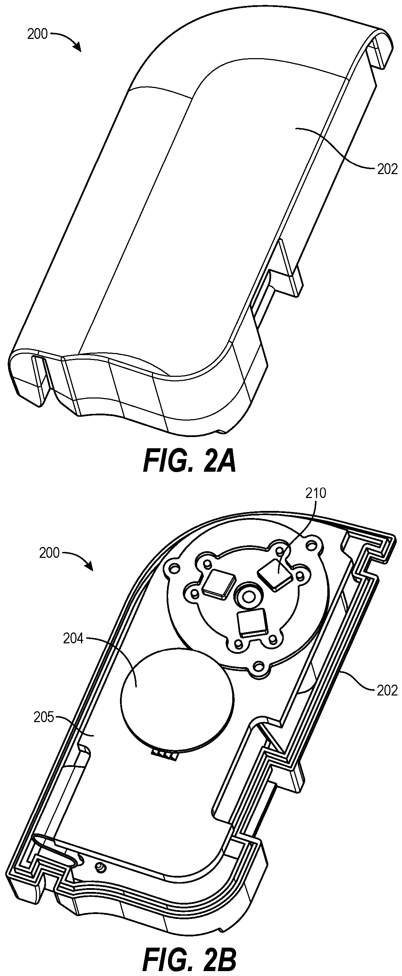

The durable assembly 200, shown in more detail in FIGS. 2A-2C, may include a housing 202, a buzzer or other alarm device 204, one or more batteries or other energy supply 206, a microprocessor (not shown), and a coil assembly 208 (which functions as a motor stator) including one or more Hall-effect sensors 210. In some examples, the energy supply 206 is a rechargeable battery, such as a rechargeable lithium battery, with enough power to drive the motor continuously without needing a capacitor or other additional energy storage device.

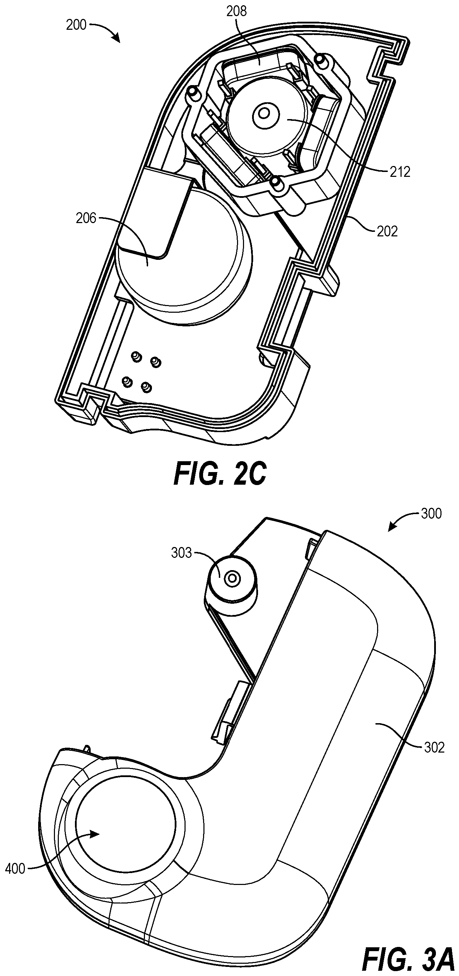

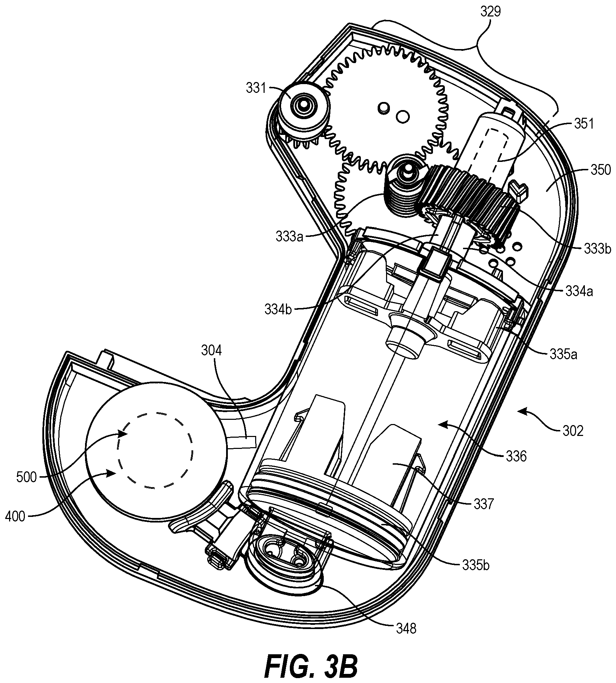

Referring now to FIGS. 2C, 3A and 3B, the coil assembly 208 can be positioned around a recessed portion 212 of the durable assembly housing 202 that is configured to fit over a protruding portion 303 of the disposable housing 302 (FIG. 3A), which in turn fits over a magnetic motor rotor 331 of the disposable assembly 300 (FIG. 3B). In this two-piece motor, the motor coil assembly 208 is in the durable assembly 200 and is positioned around the motor rotor 331 that is part of the disposable assembly 300. The Hall-effect sensors 210 (FIG. 2B) are positioned above the coil assembly 208 (FIG. 2C) in the durable assembly 200, for example, on a shelf 205 that lies within the housing 202. In this configuration, there is a gap between the motor coil assembly 208 and the motor rotor 331. Some or all of the gap may be defined by (and occupied by) housing portions, e.g., durable housing portion 212 and disposable housing portion 303 in the illustrated implementation. In other implementations, the gap between the motor coil assembly 208 and the motor rotor 331 may be occupied by only a portion of the durable assembly housing 202, or only a portion of the disposably assembly housing 302, or no structure at all and may simply be an air gap. The size of the gap, which is defined by the distance between the motor coil assembly 208 and the motor rotor 331, is typically about 0.5 mm to 2.0 mm. As such, there is no direct gear engagement or other mechanical connection between the durable assembly 200 and the disposable assembly 300. All electronics may be positioned within the durable assembly 200, with the energy needed by the disposable assembly 300 transferred by electromagnetic torque coupling, which is a coupling without direct mechanical coupling or electrical contact from the durable assembly 200. These designs afford the additional advantage of being relatively simple to make waterproof, or at least water resistant.

An exemplary motor rotor 331 may be a 2-pole, cylinder-shaped, rare earth (such as neodymium) rotor, magnetized across the diameter, with a 5 mm diameter and 5 mm height. Other suitable motor rotors may be larger or smaller, or be multi-pole. The microprocessor (not shown) directs rotation of motor rotor 331 by sequentially energizing the coils of motor coil assembly 208 to create an electromagnetic torque coupling between the motor coil assembly 208 and the motor rotor 331. The position/orientation of the rotor's poles relative to the rotating magnetic field generator (motor coil assembly 208) is measured by back EMF, a rotary encoder(s), one or more Hall-effect sensors 210, or the like. For instance, the Hall-effect sensors 210 mounted above the coil windings of the motor coil assembly 208 may be used to supply the microprocessor a count, a tachometer signal, or rotor position, enabling low-cost, closed-loop control of the rotor speed. Brushless motors of this type are efficient and run very cool.

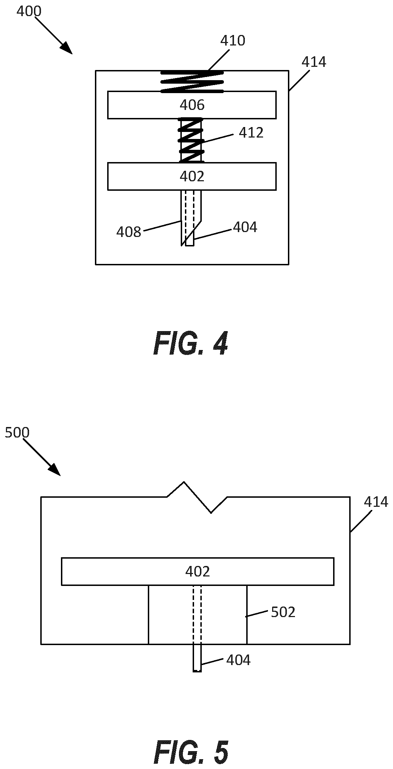

The disposable assembly 300, shown in more detail in FIGS. 3A and 3B, may include a reservoir assembly, a trigger assembly 304 (shown schematically), an insertion assembly 400, and a seal assembly 500, all mounted on a baseplate 350. The reservoir assembly can comprise a drive assembly 329, a reservoir 336, a plunger pusher 335a, and a plunger 335b. The plunger pusher 335a is coupled to the drive assembly 329, and both the plunger pusher 335a and the plunger 335b are contained within the reservoir 336. The insertion assembly 400 includes a cannula 404 (see FIG. 4) and several components for driving the cannula 404 into the user's skin. The trigger assembly 304 can operatively couple one or more components of the reservoir assembly to the insertion assembly 400 to control the timing of insertion of the cannula 404. In some examples, for example, one or more components of the trigger assembly 304 are operatively coupled to a drive assembly 329 such that remote activation of the motor moves the trigger assembly 304 into or out of engagement with the insertion assembly 400, thereby actuating the insertion assembly 400 and releasing the cannula. In some examples, insertion may be triggered locally, for example by a dial, lever, push button, etc.

Referring still to FIGS. 3A and 3B, the drive assembly 329 can comprise the magnetic motor rotor 331 and a gear train that couples the motor rotor 331 to the plunger pusher 335a (which is positioned in the reservoir 336). The magnetic motor rotor 331 may be mechanically attached through the gear train to affect translation of the plunger pusher 335a (and the plunger 335b, when attached to the plunger pusher 335a) within the reservoir 336.

As best seen in FIG. 3B, the gear train may include various gears, such as a worm drive comprised of a worm screw 333a and a worm gear 333b, and also a lead screw nut 334a and a fine-pitch lead screw 351 (enclosed by the lead screw nut 334a). The worm gear 333b is coupled to the lead screw 351 via the lead screw nut 334a. Protrusions 334b on the lead screw nut 334a correspond with recesses (not shown) inside the worm gear 333b, and a threaded portion (not shown) inside the lead screw nut 334a pairs with the thread on the lead screw 351 enclosed by the lead screw nut 334a. This configuration of the gear train prevents back-driving due to reservoir pressure, eliminating the need for a clutch or other locking mechanism. Suitable materials for the components of the gear train include, but are not limited to, stainless steel or high strength plastic, such as nylon, acetal (e.g., Delrin^{®}) or polycarbonate.

The reservoir 336 may be prefilled with a medicament. The medicament, for example, can be U-100 insulin or U-500 insulin or other concentrations of insulin to suit different user use profiles, or may be user-fillable by way of the fill port 106 (FIG. 1B). In some examples, the reservoir 336 can be mounted on a reservoir support block (not shown in FIG. 3B). A reservoir outlet fitting 348 is in fluid communication with the reservoir 336. The reservoir outlet fitting 348 can be made from a drug-compatible material, such as, but not limited to, polypropylene, cyclic olefin polymer (COP) or polyethylene.

In those cases where the reservoir 336 is filled by the user, the user may completely fill the reservoir to capacity with medicament, or the user may choose to introduce less medicament and not completely fill the reservoir. Since an unknown amount of medicament may be injected into a user-filled reservoir, a plunger-pusher zeroing procedure (or "plunger seek") may be user-initiated or may be an automatic aspect of pump operation. A plunger seek procedure precisely determines and/or sets, before any medicament dispensing, exactly how far the plunger pusher 335a travels before it engages the plunger 335b, enabling a calculation to determine the amount of medicament in the reservoir and, therefore, an estimate of time-to-empty and time for disposable assembly replacement.

The insertion assembly 400, shown schematically in FIG. 4, can include a cannula carrier 402, a cannula 404 carried by the cannula carrier 402, a needle carrier 406, a needle 408 carried by the needle carrier 406, an insertion spring 410, a retraction spring 412, and a frame 414. The insertion assembly 400 is shown in a pre-insertion state. The cannula carrier 402 and needle carrier 406 (and associated cannula 404 and needle 408) can be coupled to the frame 414 and configured to translate and/or rotate relative to the frame 414 during insertion and/or retraction of the needle 408. The needle 408, which comprises an elongate rod with a sharp distal tip, may be made of metal, such as stainless steel, or other relatively rigid biocompatible material, such as rigid plastic, ceramic, or other rigid biocompatible material, and is used to penetrate the skin and a short distance into the flesh, to make a channel for the cannula 404. The cannula 404 may be softer and/or more flexible than the needle 408, and may comprise a biocompatible polymeric material. The insertion assembly 400 can be configured to engage with the trigger assembly 304 to move (or enable movement of) the cannula carrier 402 relative to the frame 414 for insertion of the cannula 404. Activation by the trigger assembly 304 releases the insertion spring, which pushes the needle carrier 406 and cannula carrier 402 downwardly, towards the insertion site. The cannula carrier 402 may remain at the insertion site while the retraction spring 412 pushes the needle carrier 406 back upwardly, into the frame 602, thus withdrawing the needle 408 from the insertion site.

FIG. 5 schematically depicts the seal assembly 500 for maintaining a fluid-tight seal between the interior of a medicament infusion device and an exterior environment, both to prevent undesirable leakage of medicament out of the device and to prevent undesirable ingress of fluid from the patient's body into the device. The seal assembly 500 can also be configured to engage with the cannula carrier 402 such that when the cannula carrier 402 is deployed and the cannula 404 extends beyond a lower surface of the device housing and into a patient, a flowpath is established between the reservoir 336 and the cannula 404, and a fluid-tight seal is established between the cannula carrier 402 and the seal assembly 500. The seal assembly 500 can include a dock seal 502 comprising an annular sidewall configured to circumferentially surround at least a portion of the cannula carrier 402 when the cannula carrier 402 is fired. The dock seal 502 can define at least one fluid flow path therethrough that is coupled, at an inlet side, to a reservoir supply fluid flow path. The dock seal fluid flow path can be coupled, at an outlet side, to the cannula 404 (e.g., through an intervening flow path in the cannula carrier, cannula insert, or other components of the cannula carrier assembly).

### I. Selected Examples of Insertion Assemblies

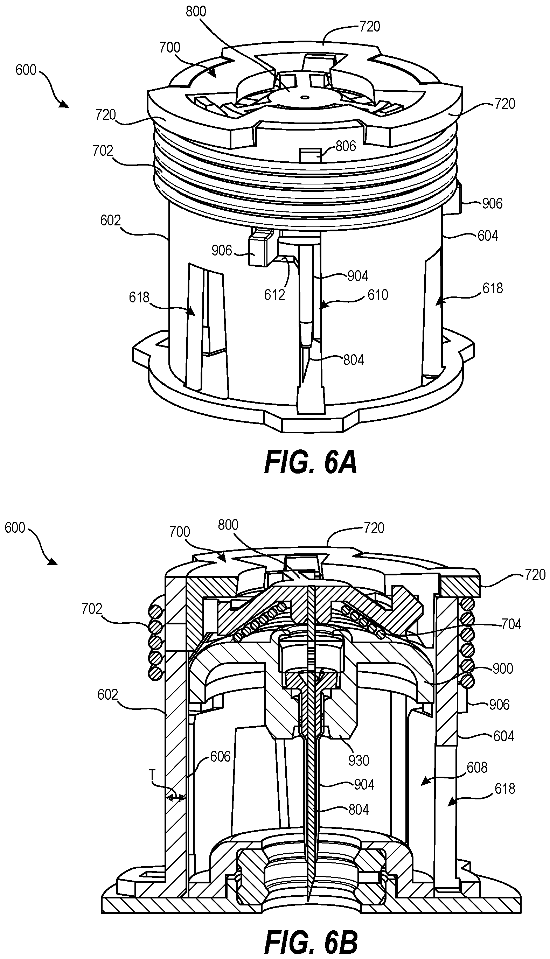

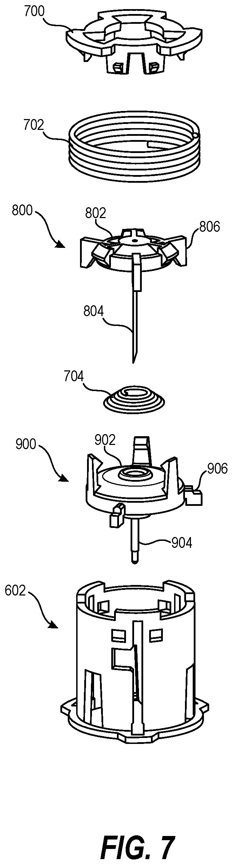

An example insertion assembly 600 for use with the device 100 of the present technology is shown in greater detail in FIGS. 6A, 6B and 7. FIGS. 6A and 6B are perspective and cross-sectional views, respectively, of the insertion assembly 600 in a first or pre-insertion state, and FIG. 7 is an exploded view showing the components of the insertion assembly 600. Referring to FIGS. 6A, 6B and 7 together, the insertion assembly 600 can comprise a cap 700, an insertion spring 702, a needle carrier 800, a needle 804 fixed to the needle carrier 800, a retraction spring 704, a cannula carrier 900, a cannula 904 fixed to the cannula carrier 900, and a frame 602.

The frame 602 can comprise a generally tubular sidewall having an outer surface 604, an inner surface 606 surrounding and defining an interior region 608 (see FIG. 6B), and a thickness T measured between the inner and outer surfaces 604, 606. The frame 602 can further include one or more longitudinal slots 610, each spanning the full thickness T of the sidewall. The cap 700 can be disposed across an opening at a top portion of the frame 602 and may include one or more ledges 720 that extend radially beyond an outer diameter of the frame 602. In some examples, the cap 700 includes a plurality of ledges 720 (as shown in FIG. 6A) spaced apart about a circumference of the insertion assembly 600, and in some examples the cap 700 includes a single ledge 720 that extends around most or all of the circumference. In any case, the insertion spring 702 may be positioned around the outer surface 604 of the frame 602, bound longitudinally between the cap ledges 720 and protrusions 906 on the cannula carrier 900 that extend through and radially beyond the slots 610 to the outer surface 604 of the frame 602. In some examples, the insertion assembly 600 does not include a cap 700 and a top portion of the frame 602 includes one or more ledges that engage a top surface of the insertion spring 702.

The needle carrier 800 can include a needle carrier body 802 and one or more needle carrier protrusions 806 extending radially away from the needle carrier body 802. Likewise, the cannula carrier 900 can include a cannula carrier body 902 and one or more cannula carrier protrusions 906 extending radially away from the cannula carrier body 902. The needle carrier body 802 and cannula carrier body 902 are disposed within the interior region 608 of the frame 602 with the needle carrier protrusions 806 and cannula carrier protrusions 906 extending into and/or through the slots 610. The slots 610 thus stabilize and guide movement of the needle carrier 800 and cannula carrier 900 during insertion, and of the needle carrier 800 during retraction. The needle carrier body 802 is positioned on top of the cannula carrier body 902 within the frame 602 with the retraction spring 704 disposed therebetween.

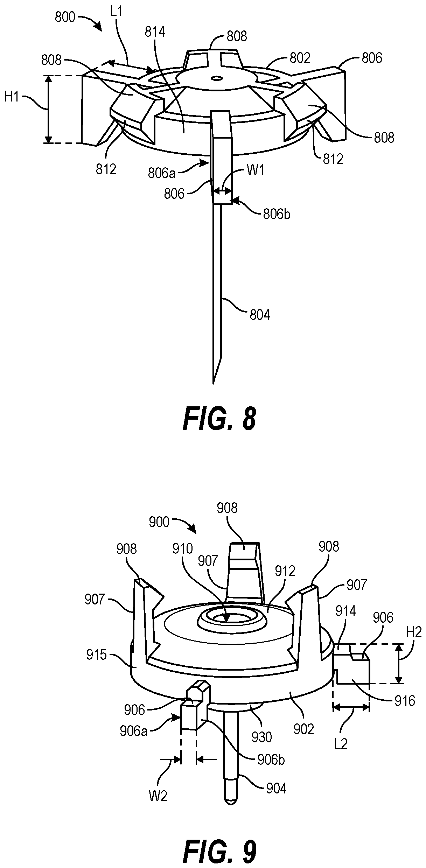

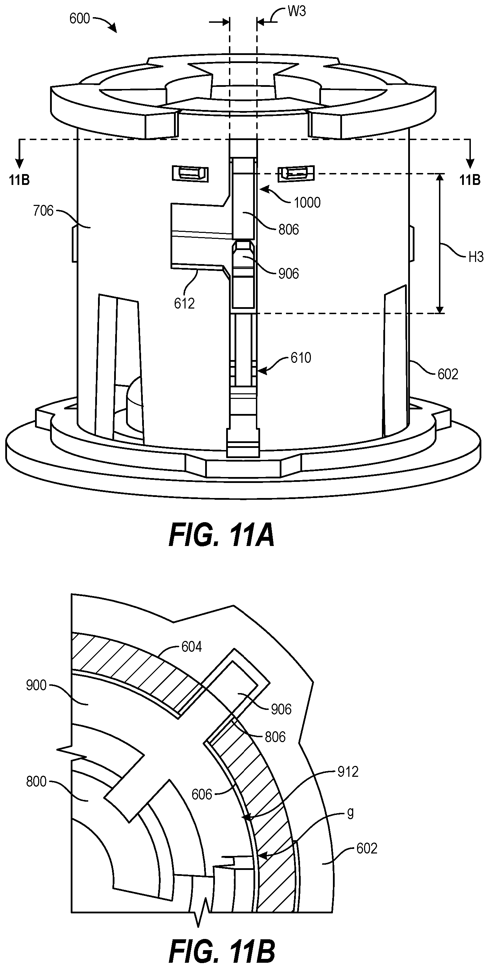

FIG. 8 is an isolated view of the needle carrier 800. The needle carrier 800 can include three needle carrier protrusions 806 spaced apart equally (i.e., 120 degrees apart) around the circumference of the needle carrier body 802. In some examples, the needle carrier 800 includes more or fewer needle carrier protrusions 806 (e.g., one needle carrier protrusion 806, two needle carrier protrusions 806, four needle carrier protrusions 806, etc.) and/or the needle carrier protrusions 806 can have different spacing. Each of the needle carrier protrusions 806 can have a length L1 measured between an outer surface 814 of the needle carrier body 802 and a distal end of the respective needle carrier protrusion 806, a height H1 between the topmost and bottommost surfaces that sit within the slot 610, lateral surfaces 806a and 806b, and a width W1 measured between the lateral surfaces 806a, 806b. At least a portion of the length L1 of each of the needle carrier protrusions 806 extend into a corresponding slot 610 in the frame 602 and the lateral surfaces 806a, 806b are configured to engage and slide along portions of the frame sidewall on either slide of the slot 610. In some examples, for example as best shown in FIGS. 6A and 11B, the distal ends of the needle carrier protrusions 806 are substantially aligned with or proximal of (radially inward of) an outer surface 604 of the frame 602 such that the needle carrier protrusions 806 do not extend radially beyond their respective slots 610.

The needle carrier 800 further includes locking ledges 808 configured to engage tabs 908 on arms 907 of the cannula carrier 900, as discussed herein. The locking ledges 808 may be spaced apart equally (i.e., 120 degrees apart) around the circumference of the needle carrier body 802 (as shown in FIG. 8), or in some examples can have different spacing. Likewise, while three locking ledges 808 are shown in FIG. 8, the needle carrier 800 may include more or fewer locking ledges 808 (e.g., one locking ledge 808, two locking ledges 808, four locking ledges 808, etc.). The radially outermost surfaces 812 of the locking ledges 808 can comprise the radially outermost surfaces of the needle carrier 800 (with the exception of the needle carrier protrusions 806) and may be spaced apart from the inner surface 606 of the frame 602 during insertion and retraction (i.e., the needle carrier body 802 is not in contact with an inner surface 606 of the frame 602). As such, the only surfaces of the needle carrier 800 that contact the frame 602 are the lateral surfaces 806a, 806b.

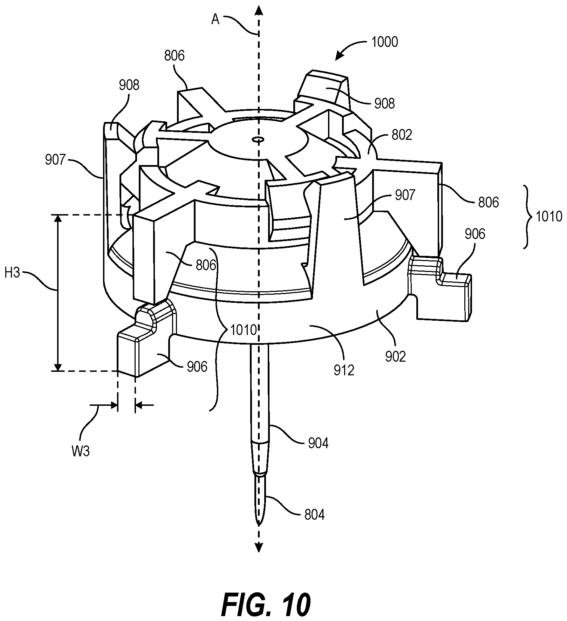

FIG. 9 is an isolated view of the cannula carrier 900. The cannula carrier 900 can include three cannula carrier protrusions 906 spaced apart equally (i.e., 120 degrees apart) around the circumference of the cannula carrier body 902. In some examples, the cannula carrier 900 includes more or fewer cannula carrier protrusions 906 (e.g., one cannula carrier protrusion 906, two cannula carrier protrusions 906, four cannula carrier protrusions 906, etc.) and/or the cannula carrier protrusions 906 can have different spacing. Each of the cannula carrier protrusions 906 can have a length L2 measured between an outer diameter of the cannula carrier body 902 and a distal end of the respective cannula carrier protrusion 906, a height H2 between the topmost and bottommost surfaces that sit within the slot 610, lateral surfaces 906a and 906b, and a width W2 measured between the lateral surfaces 906a, 906b. At least a portion of the length L2 of the respective cannula carrier protrusions 906 extend into a corresponding slot 610 in the frame 602 and the lateral surfaces 906a, 906b engage and are configured to slide along portions of the frame sidewall on either side of the slot 610. In some examples, for example as best shown in FIGS. 6A and 11B, the cannula carrier protrusions 906 extend completely through the slots 110 such that the distal ends of the cannula carrier protrusions 906 are radially farther from the insertion axis A (FIG. 10) than is the outer surface 604 of the frame 602. As such, each cannula carrier protrusion 906 includes a slot portion 914 disposed within the slot 610 and a projecting portion 916 disposed exterior of the slot 610. In some variations, for example as shown in FIGS. 6A and 6B, a bottom end of the insertion spring 702 can be supported by the top surfaces of the projecting portions 916 of the cannula carrier protrusions 906, at least when the insertion assembly 600 is in a loaded, pre-insertion state. As discussed in greater detail below, rotation of the cannula carrier 900 within the frame 602 causes the insertion spring 702 to unload, thereby pushing the cannula carrier 900 (and associated needle carrier 800) downwardly, towards the insertion site.

A radially outermost surface 915 of the cannula carrier body 902 can be spaced apart from the inner surface 606 of the frame 602 by a gap g (see FIG. 11B), and remain spaced apart during insertion and retraction (i.e., the cannula carrier body 902 is not in contact with an inner surface 606 of the frame 602). As such, the only surfaces of the cannula carrier 900 that contact the frame 602 are the lateral surfaces 906a, 906b.

The cannula carrier 900 further includes arms 907 that extend upwardly from a top portion of the cannula carrier body 902, and each of the arms 907 includes a tab 908 that extends radially inwardly from a distal end of the respective arm 907. The tabs 908 are configured to engage the locking ledges 808 of the needle carrier 800 when the needle carrier 800 and cannula carrier 900 are coupled to one another to hold the needle carrier 800 against a top surface of the cannula carrier body 902. The cannula carrier 900 may include three arms 907 spaced apart equally (i.e., 120 degrees apart) around the circumference of the cannula carrier body 902 (as shown in FIG. 9), or in some embodiments may include more or fewer arms 907 (e.g., one arm 907, two arms 907, four arms 907, etc.) and/or the arms 907 can have different spacing.

The cannula carrier body 902 may further include an opening 910 through which the needle 804 of the needle carrier 800 is received. The opening 910 can extend through a sealing portion 930 (best visualized in FIG. 6B) of the cannular carrier 900 that extends downwardly from the cannula carrier body 902. The sealing portion 930 can be configured to be received within a seal assembly at a bottom portion of the insertion assembly 600 to establish a fluid path between the reservoir and the cannula 904.

FIG. 10 shows the needle carrier 800 and cannula carrier 900 coupled to one another, for example when the insertion assembly 600 is in the pre-insertion state and during insertion of the needle 804 and cannula 904. The needle carrier 800 can be positioned on top of the cannula carrier 900 with the needle 804 disposed within a lumen of the cannula 904. The retraction spring 704 (best shown in FIG. 6B) can be loaded between a top surface of the cannula carrier 900 and a bottom surface of the needle carrier 800. As used herein, "carrier assembly 1000" refers to this temporary, coupled configuration of the needle carrier 800 and the cannula carrier 900. As previously mentioned, the tabs 908 of the arms 907 of the cannula carrier 900 may extend radially inwardly from the distal ends of the arms 907, towards the insertion axis A, to engage the corresponding locking ledges 808 on the needle carrier 800, thereby securing the needle carrier 800 to the cannula carrier 900. The fit between the locking ledges 808 and the tabs 908 may be tight enough such that the cannula carrier 900 remains engaged with the needle carrier 800 during insertion (and thereby pull the needle carrier 800 downwardly), but loose enough to allow the tabs 908 to slide circumferentially along the locking ledges 808. As discussed in greater detail below, in order to release (or unload) the insertion spring 702, the cannula carrier protrusions 906 may be rotated off a supporting ledge 612 in the frame 602 (see FIG. 6A) and into the slots 610. During rotation of the cannula carrier 900, the needle carrier 800 may remain stationary within the frame 602 so that the tabs 908 slide along the locking ledges 808.

FIG. 11A shows the insertion assembly 600 shortly after the cannula carrier protrusion 906 has been rotated off the ledge 612 and the carrier assembly 1000 begins to slide downwardly within the frame 602. In FIG. 11A, the insertion spring 702 has been removed for ease of viewing the carrier assembly 1000 within the slot 610. Prior to rotation of the cannula carrier 900, the needle carrier protrusions 806 and cannula carrier protrusions 906 are circumferentially offset about the insertion axis A (for example, as depicted in FIG. 6A). Once the cannula carrier 900 is rotated such that the cannula carrier protrusions 906 enter adjacent slots 610, the needle carrier protrusions 806 and the cannula carrier protrusions 906 become circumferentially aligned (as shown in FIG. 11A). Each circumferentially aligned pair of needle and cannula carrier protrusions 806, 906 form a sliding interface 1010 that engages a corresponding slot 610 in the frame 602 such that the slots 610 control the straightness of the insertion path of the needle 804 and cannula 904, and the straightness of the retraction path of the needle 804.

Each of the sliding interfaces 1010 can have a height H3 greater than or equal to the combined heights H1 and H2 of the associated needle carrier protrusions 806 and cannula carrier protrusions 906, respectively. The height H3 of the individual sliding interfaces 1010 will be greater than the combination of H1 and H2 when a gap exists between a bottom surface of the needle carrier protrusion 806 and the top surface of the cannula carrier protrusion 906 (for example, as shown in FIG. 10). Likewise, each of the sliding interfaces 1010 can have a width W3 that is no greater than the greater of the width W1 of the needle carrier protrusion 806 or the width W2 of the cannula carrier protrusion 906 (if W1 and W2 are different), and no less than the smaller of the width W1 of the needle carrier protrusion 806 or the width W2 of the cannula carrier protrusion 906 (if W1 and W2 are different). If W1 and W2 are the same, then each of the sliding interfaces 1010 can have a width W3 equivalent to the widths W 1, W2 of the needle carrier protrusion 806 and the cannula carrier protrusion 906.

According to several aspects of the technology, the sliding interface 1010 can have a height-to-width (H3:W3) ratio that is greater than 1, including greater than 2, greater than 3, greater than 4, greater than 5, etc. It may be beneficial to have a height-to-width (H3 :W3) ratio for the sliding interface that is greater than 1 as it reduces the risk of tilting and binding of the carrier assembly 1000 within the frame 602 during axial movement of the carrier assembly 1000 within the frame 602. By utilizing a plurality of narrow rails (e.g., the slots and protrusions), the sliding interfaces of the present technology have a relatively low effective width, thereby increasing the H:W ratio. To further increase the H:W ratio during insertion, the protrusions 806, 906 of the needle hub and the cannula carrier are vertically stacked during insertion, thereby increasing the height of the sliding interface (at least relative to a scenario where the protrusions are not stacked or the device does not include radially aligned protrusions). After the retraction mechanism is triggered and the needle carrier 800 has separated from the cannula carrier 900, the H:W ratio of the needle hub protrusions by themselves is still sufficient (e.g., greater than 1) to control the straightness of retraction.

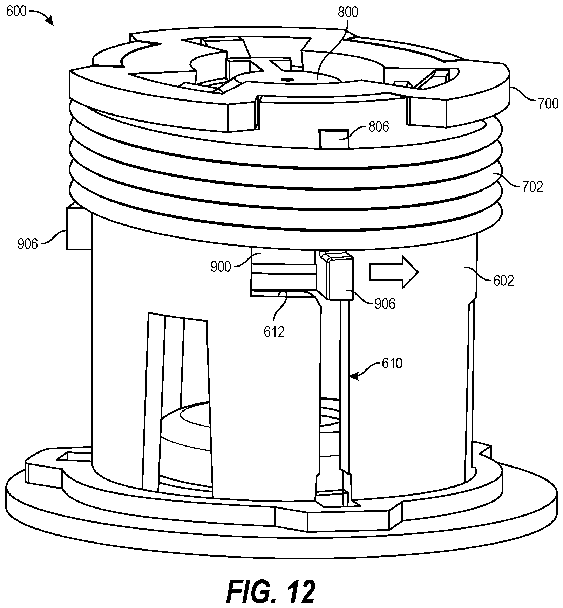

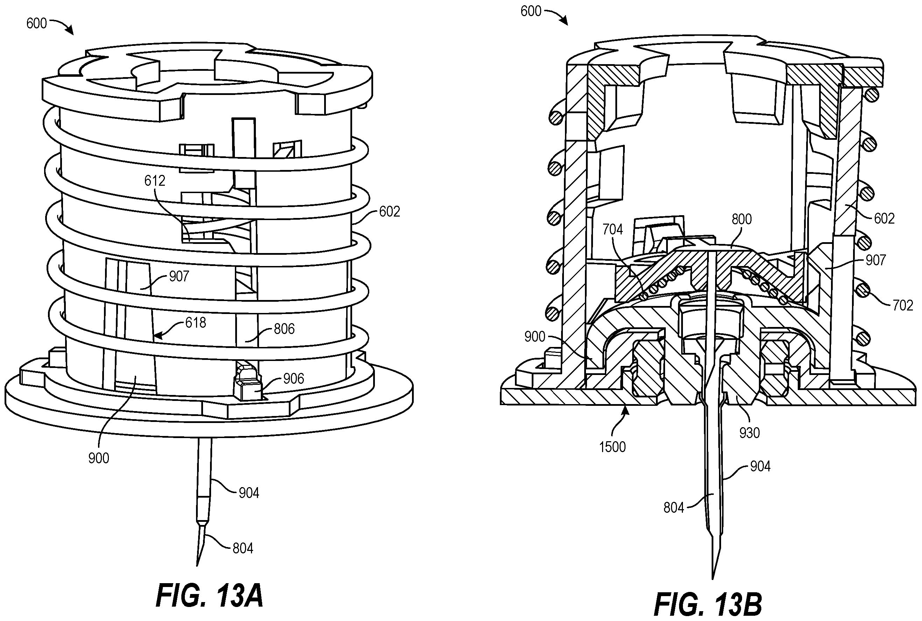

In use, the insertion assembly 600 begins in a first, pre-insertion state (shown in FIGS. 6A and 6B). In this pre-insertion configuration, the cannula carrier protrusions 906 are supported on the ledge 612 of the frame 602, which in turn holds the insertion spring 702 in a compressed (or loaded) state. Activation of a trigger assembly (such as trigger assembly 304) causes the cannula carrier 900 to rotate such that the cannula carrier protrusions 906 move away from the ledge 612 and align with the slots 610 in the frame 602, as shown in FIG. 12. While the cannula carrier 900 is shown rotating counterclockwise to release, in other examples the cannula carrier 900 may rotate clockwise to release (in such examples, the slots 610 may be disposed immediately to the left of the ledges 612). Once the cannula carrier 900 is no longer supported by the ledge 612 of the frame 602, the cannula carrier 900 can no longer resist the force of the insertion spring 702, and the elastic energy contained in the insertion spring 702 is converted to motion. The insertion spring 702 drives the carrier assembly 1000 downward, with the aligned needle carrier protrusions 806 and cannula carrier protrusions 906 sliding within their respective slots 610 in the frame 602. As the carrier assembly 1000 moves downward, the sharp distal tip of the needle 804, which extends slightly beyond the distal end of the cannula 904, penetrates the user's skin, and the cannula 904, which surrounds needle 804, is inserted so the end of the cannula 904 is about 6 mm below the surface of the user's skin. At this point, the insertion assembly 600 is in an extended configuration, as seen in FIGS. 13A and 13B.

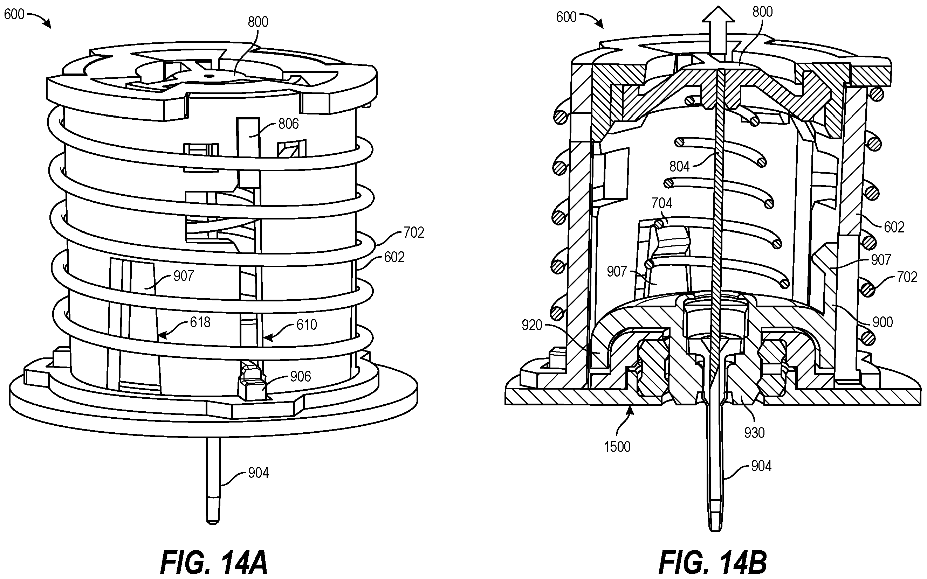

In the extended configuration, the sealing portion 930 of the cannula carrier 900 is received within a seal assembly (such as seal assembly 1500) disposed at a bottom portion of the frame 602. Also in this extended position, the arms 907 of the cannula carrier 900 align with slots 618 (FIG. 13A) in the frame 602, thereby enabling the arms 907 to bend radially away from the locking ledges 808 of the needle carrier 800 and into the slots 618. The arms 907 thus engage the slots 618 to lock the cannula carrier 900 and associated cannula 904 in place at the bottom portion of the frame 602. With the locking ledges 808 disengaged, the needle carrier 800 can no longer resist the force of the retraction spring 704, and the retraction spring 704 drives the needle carrier 800 with the attached needle 804 upward. As the needle carrier 800 moves upward, the needle carrier protrusions 806 slide within their corresponding slots 610, which prevent rotation of the needle carrier 800. Upward movement of the needle carrier 800 within the frame 602 removes the needle 804 from the user and retracts the needle 804 back into the frame 602, leaving the cannula 904 inserted, as shown in FIGS. 14A and 14B. The insertion assembly 600 remains in this retracted state while the device 100 is in use (e.g., during fluid delivery) by the user.

### II. Selected Examples of Seal Assemblies

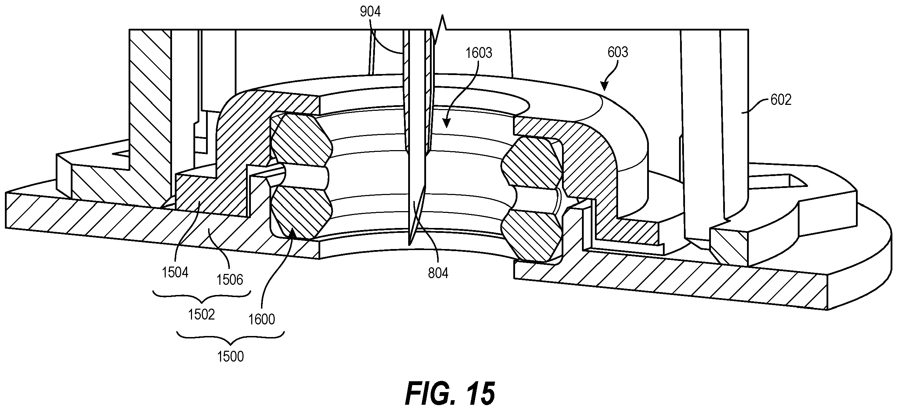

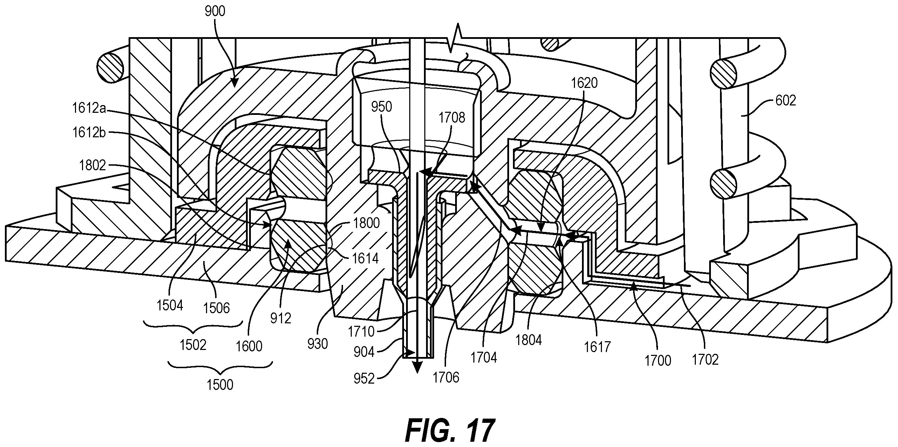

FIG. 15 is a perspective cross-sectional view of a lower portion of the insertion assembly 600 of FIGS. 6A and 6B, at which a seal assembly 1500 is disposed. The seal assembly 1500 can be configured for use with the device 100, whether device 100 is a two-piece device as described above or a single-piece, fully disposable device. The seal assembly 1500 can comprise a seal housing 1502 and a dock seal 1600 secured in position relative to the frame 602 via the seal housing 1502. When the device 100 is in use, a portion of the cannula carrier 900 is disposed within an opening 1603 extending through the dock seal 1600, and together the seal housing 1502, dock seal 1600, and cannula carrier 900 establish a fluid path between the reservoir (not shown) and the cannula 904, as discussed below with reference to FIG. 17.

In some embodiments, the seal housing 1502 includes an upper frame portion 1504 disposed within the interior region 608 of the frame 602, and a lower frame portion 1506 positioned below the frame 602, the upper frame portion 1504, and the dock seal 1600. The upper frame portion 1504 and lower frame portion 1506 mate together to secure the dock seal 1600 vertically therebetween. In some implementations, the lower frame portion 1506 defines a lower surface of the infusion device (such as infusion device 100), and may be placed in contact with a patient's skin. The seal housing 1502 and dock seal 1600 together create a fluid-tight seal between the interior of the infusion device and an exterior environment, both to prevent undesirable leakage of medicament out of the device and to prevent undesirable ingress of fluid from the patient's body, or the environment (e.g., water from a shower), into the device. In some variations, the seal housing 1502 comprises a single component, and in some implementations, the seal housing 1502 comprises more than two portions.

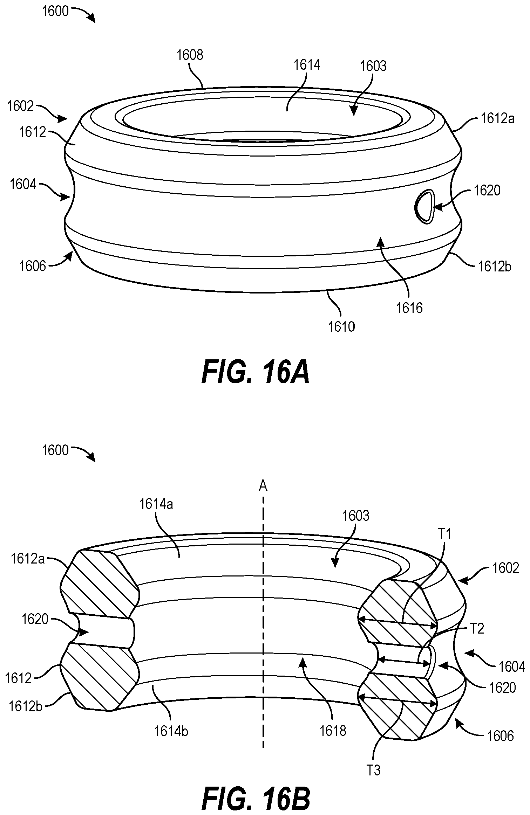

FIG. 16A is a side perspective view of the dock seal 1600 shown in FIG. 15, and FIG. 16B is a side cross-sectional view. The dock seal 1600 can comprise a generally annular sidewall having a longitudinal axis A (see FIG. 16B), a top surface 1608, a bottom surface 1610, a radially outer surface 1612, a radially inner surface 1614, and at least one transverse through-hole 1620 extending between the radially outer surface 1612 and the radially inner surface 1614. The radially inner surface 1614 can define an opening 1603 extending longitudinally through the dock seal 1600. The through-hole 1620 can define a fluid flowpath that permits fluid delivery from a reservoir (such as reservoir 336) to the cannula 904 when the cannula carrier 900 is disposed within the opening 1603 of the dock seal 1600 (as discussed below with reference to FIG. 17). In the illustrated example, the dock seal 1600 sidewall includes two opposing through-holes 1620 which are aligned with one another across the diameter of the dock seal 1600. However, in other implementations there may be only a single through-hole 1620, or there may be three, four, or more through-holes.

A thickness of the sidewall of the dock seal 1600 can vary along the longitudinal axis A such that the sidewall includes an upper widened portion 1602, a lower widened portion 1606, and a narrowed portion 1604 between the upper widened portion 1602 and the lower widened portion 1606. As seen in the cross-sectional view of FIG. 16B, the upper widened portion 1602 can have a first thickness T1, the narrowed portion 1604 can have a second thickness T2, and the lower widened portion 1606 can have a third thickness T3. In various implementations, the individual first and third thicknesses T1, T3 can be greater than the second thickness T2 such that the sidewall defines an outer annular recess 1616 at the radially outer surface 1612, and an inner annular recess 1618 at the radially inner surface 1614. The upper and lower widened portions 1602, 1606 thus define radially outermost portions that serve as sealing surfaces that contact adjacent structures to provide fluid-tight seals. These radially outermost portions include an upper radially outer sealing surface 1612a that abuts and seals against a portion of the upper frame portion 1504, a lower radially outer sealing surface 1612b that abuts and seals against a portion of the lower frame portion 1506, an upper radially inner sealing surface 1614a that abuts and seals against a region of the sealing portion 930 of the cannula carrier 900, and a lower radially inner sealing surface 1614b that abuts and seals against a region of the sealing portion 930. These abutments can provide a fluid-tight seal such that fluid that is positioned radially outside the dock seal 1600 can only pass to a radial interior of the dock seal 1600 through the through-hole 1620.

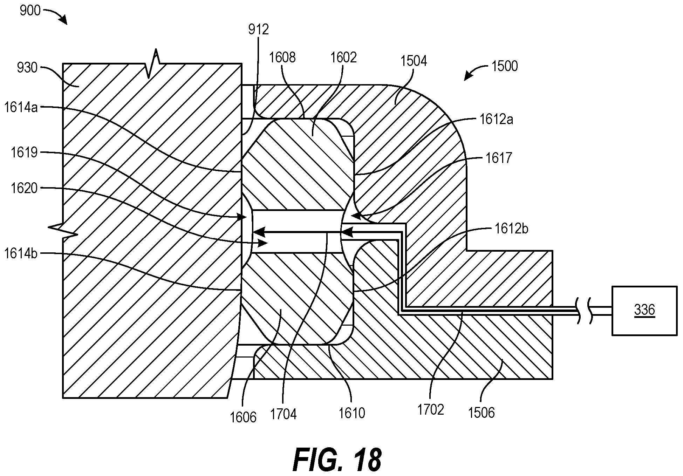

FIG. 17 is a perspective cross-sectional view of the insertion assembly of FIG. 15 in an extended position, as the needle 804 is being retracted upwardly into the interior region 608 of the frame 602. FIG. 18 is a side cross-sectional view of a portion of the seal assembly 1500 fully engaged with the cannula carrier 900. Referring to FIGS. 17 and 18 together, during normal use, the cannula carrier 900 sits within the dock seal 1600 and the cannula 904 extends distally beyond a lower surface of the seal housing 1502. In this state, the radially outer surface 912 of the sealing portion 930 of the cannula carrier 900 abuts and is sealed against the upper and lower radially inner sealing surfaces 1614a, 1614b of the dock seal 1600, the upper radially outer sealing surface 1612a of the dock seal 1600 abuts and is sealed against the upper frame portion 1504, and the lower radially outer sealing surface 1612b of the dock seal 1600 abuts and is sealed against the lower frame portion 1506. By this mating, a series of flow segments become fluidly coupled to one another to create a continuous flowpath 1700 from the reservoir 336 (shown schematically in FIG. 18) to the cannula lumen 952, such that the medicament can be delivered from the reservoir into the body of the patient. Within the seal assembly 1500 he flowpath 1700 can comprise a first flow segment 1702 that extends from the reservoir, through a space between the upper frame portion 1504 and the lower frame portion 1506, and out an opening at a radially inner surface of the seal housing 1502. A second flow segment 1704 extends through the through-hole 1620 of the dock seal 1600 and passes into a third flow segment 1706 extending through the sealing portion 930 of the cannula carrier 900. This third flow segment 1706 then leads to a fourth flow segment 1708 through a cannula insert 950 disposed within the sealing portion 930, which in turn is in fluid communication with the fifth flow segment 1710 extending through the cannula lumen 952.

The dock seal 1600 and adjacent portions of the seal housing 1502 and cannula carrier 900 together define an inner annular channel 1619 and an outer annular channel 1617, each in fluid communication with the through-hole 1620, regardless of the radial orientation of the dock seal 1600. In operation, the outer annular channel 1617 can be in fluid communication with an inlet fluid flowpath (e.g., first flow segment 1702) that carries medicament from a reservoir, and the inner annular channel 1619 can be in fluid communication with an outlet fluid flowpath (e.g., third flow segment 1706) that carries medicament through the cannula carrier 900, into the cannula lumen 952, and out of the device for delivery to a patient. The dual annular channels defined by the dock seal 1600 permit the dock seal to be inserted in the device in any rotational position while still effectively establishing a continuous fluid flowpath from the reservoir to the cannula lumen 952. For instance, even if the through-hole 1620 is offset from the inlet fluid flowpath from the reservoir by 90 degrees, fluid from the inlet fluid flowpath merely redirects along the outer annular channel 1617 until reaching the dock seal through-hole 1620, where it then passes through the through-hole 1620 and continues into the inner annular channel 1619, ultimately reaching the inlet into the cannula carrier 900. Similarly, if the dock seal through-hole 1620 is radially offset from the outlet fluid flowpath (e.g., offset with respect to a channel or through-hole in the cannula carrier, as shown in FIG. 18), fluid passing through the dock seal through-hole 1620 merely flows along the inner annular channel 1619 until reaching the inlet into the cannula carrier 900. In some implementations, a symmetrical design of the dock seal 1600 also provides benefits for manufacturing and assembly, as the dock seal can be inserted into the seal housing in any radial orientation as well as any horizontal orientation. For example, the dock seal 1600 can be radially symmetrical about axis A (as shown in FIG. 16B), and because of the annular channels, radial orientation of through-hole 1620 does not matter. In such cases, the dock seal 1600 can be inserted in any radial position and work the same. Additionally or alternatively, the dock seal 1600 can be symmetrical about a horizontal plane perpendicular to axis A, also enabling the dock seal 1600 to be installed in either orientation (top up or bottom up) and work the same.

While the dock seal and seal assembly have been described herein as forming the outer annular channel, in some embodiments the outer annular channel may be formed by the frame, and/or a combination of the dock seal and the frame.

As previously mentioned, the seal housing 1502 and dock seal 1600 together create a fluid-tight seal between the interior of the infusion device and an exterior environment. As shown in FIG. 17, a fluid ingress path is blocked by multiple seal regions, including annular seal region 1800 between the lower radially inner sealing surface 1614b of the dock seal 1600 and the radially outer surface 912 of the sealing portion 930 of the cannula carrier 900, and annular seal region 1802 between the lower radially outer sealing surface 1612b of the dock seal 1600 and a lower flange 1804 of the lower frame portion 1506 of the seal housing 1502. These contact regions provide secure seals against unwanted incursion of fluid from the surrounding environment to the interior space within the medicament infusion device.

Although the insertion assembly 600 is shown and discussed in conjunction with the seal assembly 1500, the insertion assembly 600 can be used with other seal assemblies having different components and configurations. Likewise, although the seal assembly 1500 is shown and described in conjunction with the insertion assembly 600, the seal assembly 1500 can be used with other insertion assemblies having different components and configurations.

### Conclusion

Although the devices and methods are described in the context of automatic cannula insertion and patch pumps, it should be appreciated that the techniques are equally applicable to a variety of medical devices (e.g., infusion ports) and to a variety of at least partially implantable devices (e.g., sensors). It should also be noted here that the specification describes structures and methods that are especially well-suited for the subcutaneous delivery of high concentration insulin (i.e., U-200 insulin and above) such as U-500 insulin as well as lower concentration insulin such as U-100 insulin. Nevertheless, it should be appreciated that the present inventions are applicable to a wide variety of infusion pumps and medicaments. For example, the present inventions are also applicable to medicaments such as, for example, drugs to mask pain, chemotherapy and other cancer related drugs, antibiotics, hormones, GLP-1, glucagon, various other drugs that include large molecules and proteins that may require a high level of delivery accuracy.

The descriptions of examples of the technology are not intended to be exhaustive or to limit the technology to the precise form disclosed above. Where the context permits, singular or plural terms may also include the plural or singular term, respectively. Although specific examples of, and examples for, the technology are described above for illustrative purposes, various equivalent modifications are possible within the scope of the technology, as those skilled in the relevant art will recognize. For example, while steps are presented in a given order, alternative examples may perform steps in a different order. The various examples described herein may also be combined to provide further examples.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific examples have been described herein for purposes of illustration, but that various modifications may be made without deviating from the technology. Further, while advantages associated with certain examples of the technology have been described in the context of those examples, other examples may also exhibit such advantages, and not all examples need necessarily exhibit such advantages to fall within the scope of the technology. Accordingly, the disclosure and associated technology can encompass other examples not expressly shown or described herein.

Further disclosed herein is the subject-matter of the following clauses:
1. An insertion assembly, comprising:
   a frame comprising a tubular sidewall defining an internal cavity, the frame having a first end portion configured to be positioned adjacent the skin of a user and a second end portion opposite the first end portion along a longitudinal axis of the frame, wherein the frame includes a slot extending longitudinally along a portion of the sidewall;
   a medical device configured to be inserted into the user's skin; and
   a carrier carrying the medical device and at least partially disposed within the internal cavity of the frame, the carrier axially movable within the frame from a first, cocked position proximate a second end of the frame to a second extended position proximate the first end portion of the frame,
   wherein the carrier includes a protrusion disposed within the slot of the frame and configured to slide within the slot when the carrier moves axially within the frame, and wherein:
      the protrusion is bound longitudinally between a top surface and a bottom surface, and has a height measured between the top and bottom surfaces,
      the protrusion is bound laterally between first and second side surfaces that abut the sidewall on either side of the slot, the protrusion having a width measured between the first and second side surfaces, and
      a ratio of the height of the protrusion to the width of the protrusion is greater than 1.
2. The insertion assembly of clause 1, wherein the ratio of the height of the protrusion to the width of the protrusion is at least 2.
3. The insertion assembly of clause 1 or 2, wherein the ratio of the height of the protrusion to the width of the protrusion is at least 4.
4. The insertion assembly of any one of clauses 1 to 3, wherein the carrier includes a plurality of protrusions spaced apart around a circumference of the carrier and the frame includes a plurality of slots spaced apart around a circumference of the frame, wherein each of the protrusions is disposed in a corresponding one of the slots.
5. The insertion assembly of any one of clauses 1 to 4, wherein the carrier includes a body portion disposed within the internal cavity of the frame, and wherein an outer surface of the body portion is spaced apart from an inner surface of the frame such that the body portion does not contact the inner surface of the frame when sliding axially therein.
6. The insertion assembly of any one of clauses 1 to 5, wherein the protrusion terminates at or proximal to a radially outer edge of the slot such that the protrusion does not extend radially outwardly of the slot.
7. The insertion assembly of any one of clauses 1 to 5 or of clause 6, wherein the protrusion extends completely through the slot such that a distal end of the protrusion is radially beyond the slot.
8. The insertion assembly of any one of clauses 1 to 7, wherein:
   a first portion of the protrusion terminates at or proximal to a radially outer edge of the slot such that the protrusion does not extend radially outward of the slot.
   a second portion of the protrusion extends completely through the slot such that a distal end of the protrusion is radially beyond the slot.
9. The insertion assembly of any one of clauses 1 to 8, wherein the carrier is axially movable within the frame from the second extended position to a third retracted position proximate the second end portion of the frame, and wherein a height of the protrusion as the carrier moves from the first cocked position to the second extended position is greater than a height of the protrusion as the carrier moves from the second extended position to the third retracted position.
10. An insertion assembly, comprising:
   a frame comprising a tubular sidewall defining an internal cavity, the frame having a first end portion configured to be positioned against the skin of a user and a second end portion opposite the first end portion along a longitudinal axis of the frame, wherein the frame includes a slot extending longitudinally along a portion of the sidewall;
   a needle;
   a needle carrier carrying the needle and at least partially disposed within the internal cavity of the frame, the needle carrier axially movable within the frame, wherein the needle carrier includes a needle carrier protrusion disposed within the slot of the frame and configured to slide within the slot when the needle carrier moves axially within the frame;
   a cannula;
   a cannula carrier carrying the cannula and at least partially disposed within the internal cavity of the frame, the cannula carrier axially movable within the frame, wherein the cannula carrier includes a cannula carrier protrusion disposed within the slot of the frame and configured to slide within the slot when the cannula carrier moves axially within the frame;
   wherein the cannula carrier protrusion and needle carrier protrusion together form a sliding interface, and wherein:
      the sliding interface is bound longitudinally between a top surface of the needle carrier protrusion and a bottom surface of the cannula carrier protrusion, and has a height measured between the top and bottom surfaces,
      the sliding interface is bound laterally between first and second side surfaces that abut the sidewall on either side of the slot, the sliding interface having a width measured between the first and second side surfaces, and
      a ratio of the height of the sliding interface to the width of the sliding interface is greater than 1.
11. The insertion assembly of clause 10, wherein the ratio of the height of the sliding interface to the width of the sliding interface is 2 or more.
12. The insertion assembly of clause 10 or clause 11, wherein a radial length of the cannula carrier protrusion is greater than a radial length of the needle carrier protrusion.
13. The insertion assembly of any one of clauses 10 to 12, wherein the needle carrier and the cannula carrier are coupled to one another when the insertion assembly is in a pre-insertion state, and wherein, in this pre-insertion state, the cannula carrier is configured to rotate relative to the needle carrier.
14. The insertion assembly of clause 13 or of any one of clauses 10 to 13, wherein the needle carrier and the cannula carrier are coupled to one another when the insertion assembly is in a pre-insertion state, and wherein:
   the cannula carrier includes an upwardly extending arm and a tab extending radially inwardly from a distal end portion of the arm, and
   at least in the pre-insertion state, the tab of the cannula carrier extends over and engages a locking ledge of the needle carrier, thereby securing the needle carrier to a top side of the cannula carrier.
15. The insertion assembly of clause 13 or clause 14 or of any one of clauses 10 to 14, wherein the needle carrier and cannula carrier remain coupled to one another as they move axially within the frame during insertion, and wherein the rotational positions of the needle carrier and the cannula carrier are fixed during insertion.
16. The insertion assembly of any one of clauses 13 to 15 or of any one of clauses 10 to 15, wherein the needle carrier and the cannula carrier are configured to decouple from one another once the cannula is inserted.
17. The insertion assembly of any one of clauses 13 to 16 or of any one of clauses 10 to 16, wherein the needle carrier and the cannula carrier are coupled to one another when the insertion assembly is in a pre-insertion state, and wherein:
   the cannula carrier includes an upwardly extending arm and a tab extending radially inwardly from a distal end portion of the arm,
   at least in the pre-insertion state, the tab of the cannula carrier extends over and engages a locking ledge of the needle carrier, thereby securing the needle carrier to a top side of the cannula carrier,
   wherein alignment of the arm of the cannula carrier with an opening in the frame enables the arm to move radially outwardly and disengage the locking ledge of the needle carrier, thereby releasing the needle carrier from the cannula carrier.
18. The insertion assembly of any one of clauses 10 to 17, wherein the needle carrier includes a body portion disposed within the internal cavity of the frame, and wherein an outer surface of the body portion is spaced apart from an inner surface of the frame such that the body portion does not contact the inner surface of the frame when sliding axially therein.
19. The insertion assembly of any one of clauses 10 to 18, wherein the cannula carrier includes a body portion disposed within the internal cavity of the frame, and wherein an outer surface of the body portion is spaced apart from an inner surface of the frame such that the body portion does not contact the inner surface of the frame when sliding axially therein.
20. The insertion assembly of any one of clauses 10 to 19, wherein the needle carrier protrusion and the cannula carrier protrusion are radially aligned about an insertion axis.
21. The insertion assembly of any one of clauses 10 to 20, wherein the needle carrier protrusion comprises a plurality of needle carrier protrusions spaced apart about a circumference of the needle carrier, each configured to slide within a corresponding slot in the frame.
22. The insertion assembly of any one of clauses 10 to 21, wherein the cannula carrier protrusion comprises a plurality of cannula carrier protrusions spaced apart about a circumference of the cannula carrier, each configured to slide within a corresponding slot in the frame.
23. The insertion assembly of any one of clauses 10 to 22, wherein the cannula, once inserted, is configured to deliver a medicament to the user.
24. An insertion assembly, comprising:
   a frame comprising a tubular sidewall defining an internal cavity, the frame having a first end portion configured to be positioned adjacent the skin of a user and a second end portion opposite the first end portion along a longitudinal axis of the frame, wherein the frame includes a slot extending longitudinally along a portion of the sidewall;
   a medical device configured to be inserted into the user's skin; and
   a carrier means for carrying the medical device and at least partially disposed within the internal cavity of the frame, the carrier means being axially movable within the frame from a first, cocked position proximate a second end of the frame to a second extended position proximate the first end portion of the frame,
   wherein the carrier means includes a protrusion disposed within the slot of the frame and configured to slide within the slot when the carrier means moves axially within the frame, and wherein:
      the protrusion is bound longitudinally between a top surface and a bottom surface, and has a height measured between the top and bottom surfaces,
      the protrusion is bound laterally between first and second side surfaces that abut the sidewall on either side of the slot, the protrusion having a width measured between the first and second side surfaces, and
      a ratio of the height of the protrusion to the width of the protrusion is greater than 1.
25. The insertion assembly of clause 24, wherein the ratio of the height of the protrusion to the width of the protrusion is at least 2.
26. The insertion assembly of clause 24 or 25, wherein the ratio of the height of the protrusion to the width of the protrusion is at least 4.
27. The insertion assembly of any one of clauses 24 to 26, wherein the carrier means includes a plurality of protrusions spaced apart around a circumference of the carrier means and the frame includes a plurality of slots spaced apart around a circumference of the frame, wherein each of the protrusions is disposed in a corresponding one of the slots.
28. The insertion assembly of any one of clauses 24 to 27, wherein the carrier means includes a body portion disposed within the internal cavity of the frame, and wherein an outer surface of the body portion is spaced apart from an inner surface of the frame such that the body portion does not contact the inner surface of the frame when sliding axially therein.
29. The insertion assembly of any one of clauses 24 to 28, wherein the protrusion terminates at or proximal to a radially outer edge of the slot such that the protrusion does not extend radially outwardly of the slot.
30. The insertion assembly of any one of clauses 24 to 29, wherein the protrusion extends completely through the slot such that a distal end of the protrusion is radially beyond the slot.
31. The insertion assembly of any one of clauses 24 to 30, wherein:
   a first portion of the protrusion terminates at or proximal to a radially outer edge of the slot such that the protrusion does not extend radially outward of the slot.
   a second portion of the protrusion extends completely through the slot such that a distal end of the protrusion is radially beyond the slot.
32. The insertion assembly of any one of clauses 24 to 31, wherein the carrier means is axially movable within the frame from the second extended position to a third retracted position proximate the second end portion of the frame, and wherein a height of the protrusion as the carrier means moves from the first cocked position to the second extended position is greater than a height of the protrusion as the carrier means moves from the second extended position to the third retracted position.
33. An insertion assembly, comprising:
   a frame comprising a tubular sidewall defining an internal cavity, the frame having a first end portion configured to be positioned against the skin of a user and a second end portion opposite the first end portion along a longitudinal axis of the frame, wherein the frame includes a slot extending longitudinally along a portion of the sidewall;
   a needle;
   a first carrier means for carrying the needle and at least partially disposed within the internal cavity of the frame, the first carrier means axially movable within the frame, wherein the first carrier means includes a first carrier means protrusion disposed within the slot of the frame and configured to slide within the slot when the first carrier means moves axially within the frame;
   a cannula;
   a second carrier means for carrying the cannula and at least partially disposed within the internal cavity of the frame, the second carrier means axially movable within the frame, wherein the second carrier means includes a second carrier means protrusion disposed within the slot of the frame and configured to slide within the slot when the second carrier means moves axially within the frame;
   wherein the second carrier means protrusion and first carrier means protrusion together form a sliding interface, and wherein:
      the sliding interface is bound longitudinally between a top surface of the first carrier means protrusion and a bottom surface of the second carrier means protrusion, and has a height measured between the top and bottom surfaces,
      the sliding interface is bound laterally between first and second side surfaces that abut the sidewall on either side of the slot, the sliding interface having a width measured between the first and second side surfaces, and
      a ratio of the height of the sliding interface to the width of the sliding interface is greater than 1.
34. The insertion assembly of clause 33, wherein the ratio of the height of the sliding interface to the width of the sliding interface is 2 or more.
35. The insertion assembly of clause 33 or clause 34, wherein a radial length of the second carrier means protrusion is greater than a radial length of the first carrier means protrusion.
36. The insertion assembly of any one of clauses 33 to 35, wherein the first carrier means and the second carrier means are coupled to one another when the insertion assembly is in a pre-insertion state, and wherein, in this pre-insertion state, the second carrier means is configured to rotate relative to the first carrier means.
37. The insertion assembly of clause 33 or of any one of clauses 33 to 36, wherein the first carrier means and the second carrier means are coupled to one another when the insertion assembly is in a pre-insertion state, and wherein:
   the first carrier means includes a first locking means,
   the second carrier means includes a second locking means,
   at least in the pre-insertion state, the second locking means engages the first locking means, thereby securing the first carrier means to the second carrier means.
38. The insertion assembly of clause 36 or clause 37 or of any one of clauses 33 to 37, wherein the first carrier means and second carrier means remain coupled to one another as they move axially within the frame during insertion, and wherein the rotational positions of the first carrier means and the second carrier means are fixed during insertion.
39. The insertion assembly of any one of clauses 36 to 38 or of any one of clauses 33 to 38, wherein the first carrier means and the second carrier means are configured to decouple from one another once the cannula is inserted.
40. The insertion assembly of any one of clauses 36 to 39 or of any one of clauses 33 to 39, wherein the first carrier means and the second carrier means are coupled to one another when the insertion assembly is in a pre-insertion state, and wherein:
   the first carrier means includes a first locking means,
   the second carrier means includes a second locking means,
   at least in the pre-insertion state, the second locking means engages the first locking means, thereby securing the first carrier means to the second carrier means.
   wherein alignment of the second locking means with an opening in the frame enables the second locking means to disengage the first locking means, thereby releasing the first carrier means from the second carrier means.
41. The insertion assembly of any one of clauses 33 to 40, wherein the first carrier means includes a body portion disposed within the internal cavity of the frame, and wherein an outer surface of the body portion is spaced apart from an inner surface of the frame such that the body portion does not contact the inner surface of the frame when sliding axially therein.
42. The insertion assembly of any one of clauses 33 to 41, wherein the second carrier means includes a body portion disposed within the internal cavity of the frame, and wherein an outer surface of the body portion is spaced apart from an inner surface of the frame such that the body portion does not contact the inner surface of the frame when sliding axially therein.
43. The insertion assembly of any one of clauses 33 to 42, wherein the first carrier means protrusion and the second carrier means protrusion are radially aligned about an insertion axis.
44. The insertion assembly of any one of clauses 33 to 43, wherein the first carrier means protrusion comprises a plurality of first carrier means protrusions spaced apart about a circumference of the first carrier means, each configured to slide within a corresponding slot in the frame.
45. The insertion assembly of any one of clauses 33 to 44, wherein the second carrier means protrusion comprises a plurality of second carrier means protrusions spaced apart about a circumference of the second carrier means, each configured to slide within a corresponding slot in the frame.
46. The insertion assembly of any one of clauses 33 to 45, wherein the cannula, once inserted, is configured to deliver a medicament to the user.
47. A medicament infusion device comprising:
   a reservoir assembly comprising a reservoir configured to retain medicament therein, the reservoir coupled to a reservoir outlet;
   a cannula carrier;
   an infusion cannula extending downwardly away from the cannula carrier, the cannula defining a cannula lumen and configured to be inserted into a user's skin;
   a seal assembly comprising:
      an annular dock seal configured to engage the cannula carrier such that the dock seal surrounds at least a portion of the cannula carrier, the dock seal comprising:
         an upper surface;
         a lower surface;
         a radially inner surface extending between the upper surface and the lower surface, the radially inner surface configured to abut the cannula carri er;
         a radially outer surface extending between the upper surface and the lower surface; and
         a through-hole defining a first fluid flow path between the radially inner surface and the radially outer surface, wherein the first fluid flow path is configured to be in fluid communication with the cannula lumen when the dock seal engages the cannula carrier;
      a seal housing circumferentially surrounding the dock seal, the seal housing comprising:
         an upper frame portion comprising an upper flange configured to abut the upper surface of the dock seal;
         a lower frame portion comprising a lower flange configured to abut the lower surface of the dock seal;
         an annular side portion extending between the upper frame portion and the lower frame portion, the side portion configured to abut at least a portion of the radially outer surface of the dock seal to define an annular channel between the side portion of the seal housing and the dock seal; and
         a second fluid flow path extending through the side portion and configured to be fluidically coupled to the reservoir outlet, wherein the second fluid flow path is in fluid communication with the first fluid flow path defined by the through-hole of the dock seal.
48. The medicament infusion device of clause 47, wherein the annular dock seal extends circumferentially around a vertical axis, and wherein the annular dock seal is symmetric about at a horizontal plane that is orthogonal to the vertical axis.
49. The medicament infusion device of clause 47 or clause 48, wherein contact between the radially inner surface of the dock seal and a radially outer surface of the cannula carrier defines a second annular channel, the second annular channel in fluid communication with the first fluid flow path and the cannula lumen.
50. The medicament infusion device of any one of clauses 47 to 49, wherein the dock seal comprises a variable sidewall thickness between the radially inner surface and the radially outer surface, the dock seal further comprising:
   an intermediate portion having a first sidewall thickness, wherein the through-hole extends through the intermediate portion between the radially inner surface and the radially outer surface;
   an upper widened portion disposed axially above the intermediate portion and having a second sidewall thickness greater than the first sidewall thickness, the upper widened portion defining an upper radially inner sealing surface and an upper radially outer sealing surface; and
   a lower widened portion disposed axially below the intermediate portion and having a third sidewall thickness greater than the first sidewall thickness, the lower widened portion defining a lower radially inner sealing surface and a lower radially outer sealing surface.
51. The medicament infusion device of clause 50 or of any one of clauses 47 to 50, wherein the annular channel is defined by the space between the intermediate portion of the dock seal and the side portion of the seal housing.
52. The medicament infusion device of clause 50 or clause 51 or of any one of clauses 47 to 51, wherein a second annular channel is defined by the space between the intermediate portion of the dock seal and a side portion of the cannula carrier.
53. The medicament infusion device of any one of clauses 47 to 52, wherein contact between the upper flange and the upper surface of the dock seal creates a fluid-tight seal.
54. The medicament infusion device of any one of clauses 47 to 53, wherein contact between the lower flange and the lower surface of the dock seal creates a fluid-tight seal.
55. The medicament infusion device of any one of clauses 47 to 54, wherein the cannula carrier comprises a through-hole in fluid communication with the cannula lumen and in fluid communication with the first fluid flow path.
56. The medicament infusion device of any one of clauses 47 to 55, further comprising an insertion assembly configured to move the cannula carrier between a disengaged configuration in which the cannula carrier does not contact the dock seal and an engaged configuration in which the dock seal surrounds at least a portion of the cannula carrier.
57. The medicament infusion device of any one of clauses 47 to 56, wherein a lowermost surface of the dock seal abuts the lower flange.
58. A seal assembly for a medicament infusion device, the seal assembly comprising:
   an annular dock seal comprising an upper surface, a lower surface, a radially inner surface, a radially outer surface, and a through-hole extending between the radially outer surface and the radially inner surface, the through-hole defining a first fluid flow path; and
   a seal housing at least partially surrounding the dock seal, the seal housing comprising an upper flange configured to contact the upper surface of the dock seal, a lower flange configured to contact the lower surface of the dock seal, a side portion configured to contact at least a portion of the radially outer surface of the dock seal to define an annular channel between the side portion of the seal housing and the radially outer surface of the dock seal, and a second fluid flow path extending through the side portion such that the second fluid flow path is in fluid communication with the first fluid flow path.
59. The seal assembly of clause 58, wherein the annular dock seal extends circumferentially around a vertical axis, and wherein the annular dock seal is symmetric about a horizontal plane that is orthogonal to the vertical axis.
60. The seal assembly of clause 58 or clause 59, wherein the dock seal comprises a variable sidewall thickness between the radially inner surface and the radially outer surface, the dock seal further comprising:
   an intermediate portion having a first sidewall thickness, wherein the through-hole extends through the intermediate portion between the radially inner surface and the radially outer surface;
   an upper widened portion disposed axially above the intermediate portion and having a second sidewall thickness greater than the first sidewall thickness, the upper widened portion defining an upper radially inner sealing surface and an upper radially outer sealing surface; and
   a lower widened portion disposed axially below the intermediate portion and having a third sidewall thickness greater than the first sidewall thickness, the lower widened portion defining a lower radially inner sealing surface and a lower radially outer sealing surface.
61. The seal assembly of clause 60 or of any one of clauses 58 to 60, wherein the annular channel is defined by the space between the intermediate portion of the dock seal and the side portion of the seal housing.
62. The seal assembly of clause 60 or clause 61 or of any one of clauses 58 to 61, wherein contact between the upper flange and the upper surface of the dock seal creates a fluid-tight seal, and wherein contact between the lower flange and the lower surface of the dock seal creates a fluid-tight seal.
63. The seal assembly of any one of clauses 60 to 62 or of any one of clauses 58 to 62, wherein contact between the upper flange and the upper surface of the dock seal creates a fluid-tight seal, or wherein contact between the lower flange and the lower surface of the dock seal creates a fluid-tight seal.
64. The seal assembly of any one of clauses 60 to 63 or of any one of clauses 58 to 63, wherein a lowermost surface of the dock seal abuts the lower flange.
65. A dock seal for a medicament infusion device, the dock seal comprising:
   an annular body having a sidewall defining a radially outer surface and a radially inner surface surrounding a central opening configured to receive a cannula carrier therein, wherein the sidewall has a variable thickness such that the sidewall defines:
      an intermediate portion having a first sidewall thickness;
      an upper widened portion disposed axially above the intermediate portion and having a second sidewall thickness greater than the first sidewall thickness, the upper widened portion defining an upper radially inner sealing surface and an upper radially outer sealing surface;
      a lower widened portion disposed axially below the intermediate portion and having a third sidewall thickness greater than the first sidewall thickness, the lower widened portion defining a lower radially inner sealing surface and a lower radially outer sealing surface; and
   a through-hole extending through the intermediate portion of the sidewall between the radially inner surface and the radially outer surface.
66. The dock seal of clause 65, wherein the annular body extends circumferentially around a vertical axis, and wherein the annular body is symmetric about at a horizontal plane that is orthogonal to the vertical axis.
67. The dock seal of clause 65 or clause 66, wherein the upper radially inner sealing surface and the lower radially inner sealing surface each project radially inwardly with respect to the intermediate portion.
68. The dock seal of clause 65 or of any one of clauses 65 to 67, wherein the upper radially outer sealing surface and the lower radially inner sealing surface each project radially outwardly with respect to the intermediate portion.
69. A medicament infusion device comprising:
   a reservoir assembly comprising a reservoir configured to retain medicament therein, the reservoir coupled to a reservoir outlet;
   a cannula carrying means;
   an infusion cannula extending downwardly away from the cannula carrying means, the cannula defining a cannula lumen and configured to be inserted into a user's skin;
   a seal assembly comprising:
      an annular dock seal configured to engage the cannula carrying means such that the dock seal surrounds at least a portion of the cannula carrying means, the dock seal comprising:
         an upper surface;
         a lower surface;
         a radially inner surface extending between the upper surface and the lower surface, the radially inner surface configured to abut the cannula carrying means;
         a radially outer surface extending between the upper surface and the lower surface; and
         a through-hole defining a first fluid flow path between the radially inner surface and the radially outer surface, wherein the first fluid flow path is configured to be in fluid communication with the cannula lumen when the dock seal engages the cannula carrying means;
      a seal housing circumferentially surrounding the dock seal, the seal housing comprising:
         an upper frame portion comprising an upper flange configured to abut the upper surface of the dock seal;
         a lower frame portion comprising a lower flange configured to abut the lower surface of the dock seal;
         an annular side portion extending between the upper frame portion and the lower frame portion, the side portion configured to abut at least a portion of the radially outer surface of the dock seal to define an annular channel between the side portion of the seal housing and the dock seal; and
         a second fluid flow path extending through the side portion and configured to be fluidically coupled to the reservoir outlet, wherein the second fluid flow path is in fluid communication with the first fluid flow path defined by the through-hole of the dock seal.
70. The medicament infusion device of clause 47 or of clause 69, wherein the annular dock seal extends circumferentially around a vertical axis, and wherein the annular dock seal is symmetric about at a horizontal plane that is orthogonal to the vertical axis.
71. The medicament infusion device of clause 47 or clause 48 or of clause 69 or of clause 70, wherein contact between the radially inner surface of the dock seal and a radially outer surface of the cannula carrying means defines a second annular channel, the second annular channel in fluid communication with the first fluid flow path and the cannula lumen.
72. The medicament infusion device of any one of clauses 47 to 49 or of any one of clauses 69 to 71, wherein the dock seal comprises a variable sidewall thickness between the radially inner surface and the radially outer surface, the dock seal further comprising:
   an intermediate portion having a first sidewall thickness, wherein the through-hole extends through the intermediate portion between the radially inner surface and the radially outer surface;
   an upper widened portion disposed axially above the intermediate portion and having a second sidewall thickness greater than the first sidewall thickness, the upper widened portion defining an upper radially inner sealing surface and an upper radially outer sealing surface; and
   a lower widened portion disposed axially below the intermediate portion and having a third sidewall thickness greater than the first sidewall thickness, the lower widened portion defining a lower radially inner sealing surface and a lower radially outer sealing surface.
73. The medicament infusion device of clause 50 or of any one of clauses 69 to 72, wherein the annular channel is defined by the space between the intermediate portion of the dock seal and the side portion of the seal housing.
74. The medicament infusion device of clause 50 or clause 51 or of any one of clauses 69 to 73, wherein a second annular channel is defined by the space between the intermediate portion of the dock seal and a side portion of the cannula carrying means.
75. The medicament infusion device of any one of clauses 47 to 52 or of any one of clauses 69 to 74, wherein contact between the upper flange and the upper surface of the dock seal creates a fluid-tight seal.
76. The medicament infusion device of any one of clauses 47 to 53 or of any one of clauses 69 to 75, wherein contact between the lower flange and the lower surface of the dock seal creates a fluid-tight seal.
77. The medicament infusion device of any one of clauses 47 to 54 or of any one of clauses 69 to 76, wherein the cannula carrying means comprises a through-hole in fluid communication with the cannula lumen and in fluid communication with the first fluid flow path.
78. The medicament infusion device of any one of clauses 47 to 55 or of any one of clauses 69 to 77, further comprising an insertion assembly configured to move the cannula carrying means between a disengaged configuration in which the cannula carrying means does not contact the dock seal and an engaged configuration in which the dock seal surrounds at least a portion of the cannula carrying means.
79. The medicament infusion device of any one of clauses 47 to 56 or of any one of clauses 69 to 78, wherein a lowermost surface of the dock seal abuts the lower flange.
80. A seal assembly for a medicament infusion device, the seal assembly comprising:
   an annular dock seal comprising an upper surface, a lower surface, a radially inner surface, a radially outer surface, and a through-hole extending between the radially outer surface and the radially inner surface, the through-hole defining a first fluid flow path; and
   a seal housing at least partially surrounding the dock seal, the seal housing comprising an upper flange configured to contact the upper surface of the dock seal, a lower flange configured to contact the lower surface of the dock seal, a side portion configured to contact at least a portion of the radially outer surface of the dock seal to define an annular channel between the side portion of the seal housing and the radially outer surface of the dock seal, and a second fluid flow path extending through the side portion such that the second fluid flow path is in fluid communication with the first fluid flow path.
81. The seal assembly of clause 58 or of clause 80, wherein the annular dock seal extends circumferentially around a vertical axis, and wherein the annular dock seal is symmetric about a horizontal plane that is orthogonal to the vertical axis.
82. The seal assembly of clause 58 or clause 59 or clause 80 or clause 81, wherein the dock seal comprises a variable sidewall thickness between the radially inner surface and the radially outer surface, the dock seal further comprising:
   an intermediate portion having a first sidewall thickness, wherein the through-hole extends through the intermediate portion between the radially inner surface and the radially outer surface;
   an upper widened portion disposed axially above the intermediate portion and having a second sidewall thickness greater than the first sidewall thickness, the upper widened portion defining an upper radially inner sealing surface and an upper radially outer sealing surface; and
   a lower widened portion disposed axially below the intermediate portion and having a third sidewall thickness greater than the first sidewall thickness, the lower widened portion defining a lower radially inner sealing surface and a lower radially outer sealing surface.
83. The seal assembly of clause 60 or of any one of clauses 80 to 82, wherein the annular channel is defined by the space between the intermediate portion of the dock seal and the side portion of the seal housing.
84. The seal assembly of clause 60 or clause 61 or of any one of clauses 80 to 83, wherein contact between the upper flange and the upper surface of the dock seal creates a fluid-tight seal, and wherein contact between the lower flange and the lower surface of the dock seal creates a fluid-tight seal.
85. The seal assembly of any one of clauses 60 to 62 or of any one of clauses 80 to 84, wherein contact between the upper flange and the upper surface of the dock seal creates a fluid-tight seal, and wherein contact between the lower flange and the lower surface of the dock seal creates a fluid-tight seal.
86. The seal assembly of any one of clauses 60 to 63 or of any one of clauses 80 to 85, wherein a lowermost surface of the dock seal abuts the lower flange.
87. A dock seal for a medicament infusion device, the dock seal comprising:
   an annular body having a sidewall defining a radially outer surface and a radially inner surface surrounding a central opening configured to receive a cannula carrying means therein, wherein the sidewall has a variable thickness such that the sidewall defines:
      an intermediate portion having a first sidewall thickness;
      an upper widened portion disposed axially above the intermediate portion and having a second sidewall thickness greater than the first sidewall thickness, the upper widened portion defining an upper radially inner sealing surface and an upper radially outer sealing surface;
      a lower widened portion disposed axially below the intermediate portion and having a third sidewall thickness greater than the first sidewall thickness, the lower widened portion defining a lower radially inner sealing surface and a lower radially outer sealing surface; and
   a through-hole extending through the intermediate portion of the sidewall between the radially inner surface and the radially outer surface.
88. The dock seal of clause 65 or clause 87, wherein the annular body extends circumferentially around a vertical axis, and wherein the annular body is symmetric about at a horizontal plane that is orthogonal to the vertical axis.
89. The dock seal of clause 65 or clause 87 or clause 88, wherein the upper radially inner sealing surface and the lower radially inner sealing surface each project radially inwardly with respect to the intermediate portion.
90. The dock seal of clause 65 or of any one of clauses 87 to 89, wherein the upper radially outer sealing surface and the lower radially inner sealing surface each project radially outwardly with respect to the intermediate portion.

## Claims

1. An insertion assembly, comprising:
a frame comprising a tubular sidewall defining an internal cavity, the frame having a first end portion configured to be positioned adjacent the skin of a user and a second end portion opposite the first end portion along a longitudinal axis of the frame, wherein the frame includes a slot extending longitudinally along a portion of the sidewall;
a medical device configured to be inserted into the user's skin; and
a carrier carrying the medical device and at least partially disposed within the internal cavity of the frame, the carrier axially movable within the frame from a first, cocked position proximate a second end of the frame to a second extended position proximate the first end portion of the frame,
wherein the carrier includes a protrusion disposed within the slot of the frame and configured to slide within the slot when the carrier moves axially within the frame, and wherein:
the protrusion is bound longitudinally between a top surface and a bottom surface, and has a height measured between the top and bottom surfaces,
the protrusion is bound laterally between first and second side surfaces that abut the sidewall on either side of the slot, the protrusion having a width measured between the first and second side surfaces, and
a ratio of the height of the protrusion to the width of the protrusion is greater than 1.

2. The insertion assembly of Claim 1, wherein the ratio of the height of the protrusion to the width of the protrusion is at least 2, and/or wherein the ratio of the height of the protrusion to the width of the protrusion is at least 4.

3. The insertion assembly of any one of Claims 1 to 2, wherein the carrier includes a plurality of protrusions spaced apart around a circumference of the carrier and the frame includes a plurality of slots spaced apart around a circumference of the frame, wherein each of the protrusions is disposed in a corresponding one of the slots.

4. The insertion assembly of any one of Claims 1 to 3, wherein the carrier includes a body portion disposed within the internal cavity of the frame, and wherein an outer surface of the body portion is spaced apart from an inner surface of the frame such that the body portion does not contact the inner surface of the frame when sliding axially therein.

5. The insertion assembly of any one of Claims 1 to 4, wherein the protrusion terminates at or proximal to a radially outer edge of the slot such that the protrusion does not extend radially outwardly of the slot, or
wherein the protrusion extends completely through the slot such that a distal end of the protrusion is radially beyond the slot.

6. The insertion assembly of any one of Claims 1 to 5, wherein:
a first portion of the protrusion terminates at or proximal to a radially outer edge of the slot such that the protrusion does not extend radially outward of the slot.
a second portion of the protrusion extends completely through the slot such that a distal end of the protrusion is radially beyond the slot.

7. The insertion assembly of any one of Claims 1 to 6, wherein the carrier is axially movable within the frame from the second extended position to a third retracted position proximate the second end portion of the frame, and wherein a height of the protrusion as the carrier moves from the first cocked position to the second extended position is greater than a height of the protrusion as the carrier moves from the second extended position to the third retracted position.

8. An insertion assembly, comprising:
a frame comprising a tubular sidewall defining an internal cavity, the frame having a first end portion configured to be positioned against the skin of a user and a second end portion opposite the first end portion along a longitudinal axis of the frame, wherein the frame includes a slot extending longitudinally along a portion of the sidewall;
a needle;
a needle carrier carrying the needle and at least partially disposed within the internal cavity of the frame, the needle carrier axially movable within the frame, wherein the needle carrier includes a needle carrier protrusion disposed within the slot of the frame and configured to slide within the slot when the needle carrier moves axially within the frame;
a cannula;
a cannula carrier carrying the cannula and at least partially disposed within the internal cavity of the frame, the cannula carrier axially movable within the frame, wherein the cannula carrier includes a cannula carrier protrusion disposed within the slot of the frame and configured to slide within the slot when the cannula carrier moves axially within the frame;
wherein the cannula carrier protrusion and needle carrier protrusion together form a sliding interface, and wherein:
the sliding interface is bound longitudinally between a top surface of the needle carrier protrusion and a bottom surface of the cannula carrier protrusion, and has a height measured between the top and bottom surfaces,
the sliding interface is bound laterally between first and second side surfaces that abut the sidewall on either side of the slot, the sliding interface having a width measured between the first and second side surfaces, and
a ratio of the height of the sliding interface to the width of the sliding interface is greater than 1.

9. The insertion assembly of Claim 8, wherein the ratio of the height of the sliding interface to the width of the sliding interface is 2 or more, and/or
wherein a radial length of the cannula carrier protrusion is greater than a radial length of the needle carrier protrusion.

10. The insertion assembly of any one of Claims 8 to 9, wherein the needle carrier and the cannula carrier are coupled to one another when the insertion assembly is in a pre-insertion state, and wherein, in this pre-insertion state, the cannula carrier is configured to rotate relative to the needle carrier.

11. The insertion assembly of any one of Claims 8 to 10, wherein the needle carrier and the cannula carrier are coupled to one another when the insertion assembly is in a pre-insertion state, and wherein:
the cannula carrier includes an upwardly extending arm and a tab extending radially inwardly from a distal end portion of the arm, and
at least in the pre-insertion state, the tab of the cannula carrier extends over and engages a locking ledge of the needle carrier, thereby securing the needle carrier to a top side of the cannula carrier.

12. The insertion assembly of any one of Claims 8 to 11, wherein the needle carrier and cannula carrier remain coupled to one another as they move axially within the frame during insertion, and wherein the rotational positions of the needle carrier and the cannula carrier are fixed during insertion, and/or
wherein the needle carrier and the cannula carrier are configured to decouple from one another once the cannula is inserted.

13. The insertion assembly of any one of Claims 8 to 12, wherein the needle carrier and the cannula carrier are coupled to one another when the insertion assembly is in a pre-insertion state, and wherein:
the cannula carrier includes an upwardly extending arm and a tab extending radially inwardly from a distal end portion of the arm,
at least in the pre-insertion state, the tab of the cannula carrier extends over and engages a locking ledge of the needle carrier, thereby securing the needle carrier to a top side of the cannula carrier,
wherein alignment of the arm of the cannula carrier with an opening in the frame enables the arm to move radially outwardly and disengage the locking ledge of the needle carrier, thereby releasing the needle carrier from the cannula carrier.

14. The insertion assembly of any one of Claims 8 to 13, wherein the needle carrier includes a body portion disposed within the internal cavity of the frame, and wherein an outer surface of the body portion is spaced apart from an inner surface of the frame such that the body portion does not contact the inner surface of the frame when sliding axially therein, and/or
wherein the cannula carrier includes a body portion disposed within the internal cavity of the frame, and wherein an outer surface of the body portion is spaced apart from an inner surface of the frame such that the body portion does not contact the inner surface of the frame when sliding axially therein.

15. The insertion assembly of any one of Claims 8 to 14, wherein the needle carrier protrusion and the cannula carrier protrusion are radially aligned about an insertion axis, and/or
wherein the needle carrier protrusion comprises a plurality of needle carrier protrusions spaced apart about a circumference of the needle carrier, each configured to slide within a corresponding slot in the frame, and/or
wherein the cannula carrier protrusion comprises a plurality of cannula carrier protrusions spaced apart about a circumference of the cannula carrier, each configured to slide within a corresponding slot in the frame, and/or
wherein the cannula, once inserted, is configured to deliver a medicament to the user.
